# EUROPÄISCHE PATENTSCHRIFT

(11) **EP 4 114 332 B1**
(45) Veröffentlichungstag und Bekanntmachung des Hinweises auf die Patenterteilung: **01.05.2024**
(21) Anmeldenummer: 21710458.7
(22) Anmeldetag: 05.03.2021
(51) Int. Cl.: A61F 13/34, B65H 69/04

(54) **VORRICHTUNG UND VERFAHREN ZUR VERKNOTUNG EINES FADENENDES**
DEVICE AND METHOD FOR KNOTTING A STRING END
DISPOSITIF ET PROCÉDÉ DE NOUAGE D'UNE EXTRÉMITÉ DE CORDON

(30) Priorität: 05.03.2020 CH 2632020
(43) Veröffentlichungstag der Anmeldung: 11.01.2023
(73) Patentinhaber: Ruggli AG, 5322 Koblenz (CH)
(72) Erfinder: SCHULER, Samuel, 4051 Basel (CH)
(74) Vertreter: IPrime Rentsch Kaelin AG
(86) Internationale Anmeldenummer: PCT/EP2021/055636
(87) Internationale Veröffentlichungsnummer: WO 2021/176077

(56) Entgegenhaltungen:
- WO-A1-2016/207242
- WO-A1-2017/115337
- CH-A1- 714 846

## Beschreibung

Die vorliegende Erfindung betrifft eine Vorrichtung zur Verknotung eines Fadenendes, sodass eine Schlaufe gebildet wird. Sie betrifft insbesondere eine Vorrichtung zur Verknotung eines Fadenendes eines Rückholfadens für Tampons.

Die Erfindung betrifft weiter ein Verfahren zur Verknotung eines Fadenendes, sowie eine Vorrichtung zur Herstellung von Tampons mit einem proximalen Rückholfaden, umfassend zwei miteinander verknüpfte Fadenenden, alles jeweils gemäss den Oberbegriffen der unabhängigen Patentansprüche.

### Technologischer Hintergrund

Tampons dienen der monatlichen Frauenhygiene und sind in der Regel aufgerollte oder zusammengefaltete Bahnen saugfähigen Materials, die verdichtet werden. Gerollte Tampons umfassen in der Regel eine oder mehrere Schichten saugfähigen Materials, sowie allfällige zusätzliche nicht gewobene Schichten und werden von einem Ende her längs aufgerollt. Moderne Herstellungsverfahren sehen zusätzlich die Ausbildung eines konisch zulaufenden distalen Kopfendes vor. Gefaltete Tampons, sog. "Teabag-Tampons", werden in einem Zickzack ähnlich einer Ziehharmonika gefaltet und allenfalls ebenfalls wie die aufgerollten Tampons in einem Formungsschritt mit einem konisch zulaufenden Kopfende versehen. Ungeachtet der Herstellungsweise bedürfen alle Tampons eines Ausziehmittels, um nach Gebrauch wieder aus der Körperöffnung entfernt zu werden.

Dabei ist es besonders wichtig, dass das Auszugsmittel auf keinen Fall abreissen darf, resp. die Befestigung des Auszugsmittels am Tampon, d.h. am saugfähigen Material oder Laminat, nicht abreissen darf. Aus diesem Grund werden Bänder oder Fäden benutzt, die quer zur Längsrichtung des Bandes solcher aufgerollter, resp. gefalteter Tampons um diese gelegt werden, sodass das Auszugsmittel eine Schleife quer zur Längsausdehnung des Bandes bildet. Nach erfolgtem Aufrollen, resp. Falten kommt die Schlaufe vorzugsweise vollständig im Innern des Tampons zum Liegen, und ein Abreissen des Auszugsmittels vom saugfähigen Material ist praktisch ausgeschlossen, da durch den Auszug die Auszugskraft nicht nur an einem proximalen Ende des Tampons wirkt, sondern durch die Schlaufe auch das distale Ende hinausgezogen wird.

In der Regel sehen die Herstellungsprozesse für solche Tampons vor, dass ein Rückholfaden vor dem Wickeln, resp. Zusammenfalten des Tampons um das bandförmige Material gelegt wird und ebenfalls vorgängig verknotet wird. Die WO 2016/207242 (Ruggli Projects AG, Hagendorn-CH) zeigt ein Herstellungsverfahren für einen Tampon, bei dem insbesondere ein zusätzliches Problem von gewickelten Tampons gelöst wird. Durch das Umlegen der Schlaufe um das bandförmige Material wirkt nämlich die Auszugskraft bei Bedienen des Rückziehfadens am distalen Ende, also am Kopfende des Tampons, es kann aber vorkommen, dass dabei der aufgerollte Tampon teleskopartig verlängert und ausgezogen wird. Dieses Dokument zeigt, wie ein solches Teleskopieren mittels eines Laminatstreifens im Wesentlichen verhindert wird.

Die Tamponherstellungsmaschinen der neuesten Generation arbeiten allerdings mit weitaus höheren Stückzahlen als die bisherigen. Die Schweizer Patentanmeldung mit der Nr. 00426/18 (Ruggli Projects AG, Hagendorn-CH) zeigt eine Vorrichtung zur Formung von Tampons aus bandförmigem Material, bei dem ein Rückholfaden um einen Streifen eines bandförmigen Materials gelegt wird und vor dem Wickeln, welches in dieser Veröffentlichung beschrieben wird, verknotet wird. Die darin gezeigte Vorrichtung vermag mehr als 140 Tampons pro Minute zu verarbeiten. Bestehende Verknotungsmaschinen sind diesen Stückzahlen nicht mehr gewachsen.

WO 2017/115337 offenbart ein Verfahren zum Aufwickeln von Tampons.

Es besteht somit ein Bedürfnis nach Verknotungsmaschinen, welche in der Lage sind, hohe Stückzahlen an Knoten pro Zeiteinheit herzustellen, ohne dabei Qualitätseinbussen in Kauf zu nehmen.

### Darstellung der Erfindung

Es ist somit eine Aufgabe der vorliegenden Erfindung, bestehende Verknotungsvorrichtungen zu verbessern und für die Bearbeitung von höheren Stückzahlen geeignet zu machen. Insbesondere ist es eine Aufgabe der vorliegenden Erfindung, eine Vorrichtung zur Verknotung von Fadenenden bereitzustellen, welche effizient ist und einen geringeren Wartungsaufwand aufweisen.

Es ist eine weitere besondere Aufgabe der vorliegenden Erfindung, eine Vorrichtung zur Verknüpfung von Fadenenden bereitzustellen, welche mindestens ein Problem des Bekannten löst.

Diese Aufgabe wurde mit einer Vorrichtung zur Verknotung eines Fadenendes, sodass eine Schlaufe gebildet wird, gelöst, sowie mit einem entsprechenden Verfahren und einer Vorrichtung zur Herstellung von Tampons mit einem proximalen Rückholfaden, jeweils gemäss kennzeichnendem Teil der unabhängigen Ansprüche.

Ein Aspekt der vorliegenden Erfindung betrifft eine Vorrichtung zur Verknotung eines Fadenendes, sodass eine Schlaufe gebildet wird. Bevorzugt handelt es sich dabei um das Fadenende eines Rückholfadens für Tampons.

Die Vorrichtung umfasst ein Knoter-Aggregat zur Verknotung von zwei Fadenstrecken des Fadenendes. Sie umfasst weiter einen Mitnehmer, zum Zuführen der Fadenstrecken an das Knoter-Aggregat. Die erfindungsgemässe Vorrichtung umfasst eine Umlaufbahn, entlang welcher der Mitnehmer um das Knoter-Aggregat geführt wird. Die Umlaufbahn weist einen Beschleunigungsbereich auf, in dem der Mitnehmer auf eine erste Geschwindigkeit beschleunigbar ist. Die Umlaufbahn weist weiter einen Bremsbereich auf, in dem der Mitnehmer auf eine zweite Geschwindigkeit bremsbar ist. Die erfindungsgemässe Vorrichtung umfasst eine als Klinke ausgebildete Haltevorrichtung, die ausgelegt ist eine Rückstellkraft auf ein Schlaufenende des zu verknotenden Fadenendes auszuüben.

Obschon die vorliegende Erfindung vorgängig im Kontext von Tampons zur Verwendung bei der monatlichen Frauenhygiene beschrieben wird, so versteht es sich für einen Fachmann von selbst, dass sämtliche Arten von Tampons, welche einen Rückholfaden aufweisen, von der Lehre der vorliegenden Erfindung profitieren können, wie z.B. auch chirurgische Tampons zum Verschliessen von Blutgefässen, Wunden und Analtampons, die vorwiegend im medizinischen Umfeld verwendet werden.

In einer besonderen Ausführungsform ist der Mitnehmer ausgelegt, um ein Fadenende auf der Umlaufbahn zu führen und insbesondere von einer Fadenspule abzuspulen. Dazu kann der Mitnehmer z.B. mit einem Greifer und/oder einer Klemme ausgestattet sein, welcher eine Fadenstrecke des Fadenendes zu greifen vermag. Alternativ zu einem Greifer kann der Mitnehmer mit einer Spule oder Rolle versehen sein, über die das Fadenende geführt ist.

Im Sinne der vorliegenden Erfindung kann der Mitnehmer als bremsbar, resp. beschleunigbar angesehen werden, wenn er auf seiner Strecke z.B. mit einem Antriebsmittel wirkverbunden verbunden ist, welches brems- und/oder beschleunigbar ist.

In einer besonderen Ausführungsform kann der Mitnehmer, z.B. mittels eines Stiftes, in eine angetriebene Kette eingreifen, welche auf der Umlaufbahn gelagert ist. Diese Kette kann mittels eines Antriebs auf eine Geschwindigkeit beschleunigt werden und bei Bedarf gebremst werden. Der in die Kette eingreifende Mitnehmer würde ebenfalls beschleunigt, resp. gebremst werden.

In einer alternativen Ausführungsform könnte der Mitnehmer starr mit einem beweglichen an der Umlaufbahn gelagerten Schlitten befestigt sein, der wiederum entlang der Umlaufbahn beschleunigt, resp. gebremst wird, sodass sich die Beschleunigung und Bremswirkung auf den Mitnehmer auswirkt.

In einer weiteren alternativen Ausführungsform kann der Mitnehmer über einen Riemen angetrieben sein, welcher entlang auf der Umlaufbahn beschleunigt, resp. gebremst werden kann. Ebenfalls denkbar wäre ein magnetischer Umlaufantrieb, bei dem erregbare Magnete entlang der Umlaufbahn einen auf einer Umlaufbahn gelagerten Mitnehmer zu beschleunigen, respektive zu bremsen vermögen.

In einer besonderen Ausführungsform umfasst die Umlaufbahn ein Ringlager und ist selbst um eine zentrale Einheit drehbar gelagert. Der Mitnehmer ist dann zum Beispiel mit der drehbar gelagerten Umlaufbahn starr verbunden. Die Umlaufbahn kann angetrieben werden, indem z.B. eine Verzahnung an der Umlaufbahn vorgesehen ist, welche mittels eines Antriebs, z.B. über einen Zahnriemen, antreibbar und beschleunigbar, resp. bremsbar ist.

In einer besonderen Ausführungsform kann der Mitnehmer direkt angetrieben sein. Bevorzugt weist die erfindungsgemässe Vorrichtung einen Direktantrieb für den Mitnehmer aus. Dieser Direktantrieb kann in einer weiteren besonderen Ausführungsform als elektrischer Direktantrieb ausgestaltet sein, insbesondere als Torqueantrieb ausgestalten. Dazu kann zum Beispiel aussen oder innen an der Umlaufbahn ein Stator vorgesehen sein und der Mitnehmer als Rotor.

Im Sinne der vorliegenden Erfindung kann eine Antriebssteuerung vorgesehen sein, welche ausgelegt ist, einen Beschleunigungsbereich und einen Bremsbereich für die Umlaufbahn zu definieren. Die Antriebssteuerung könnte ausgelegt sein, um sicherzustellen, dass der Mitnehmer die Umlaufbahn in einer Umdrehung mit mindestens zwei verschiedenen Geschwindigkeiten durchläuft, wobei zum Beispiel die verschiedenen Geschwindigkeiten von einem Bremsbereich, resp. einem Beschleunigungsbereich eingeläutet werden.

In einer weiteren besonderen Ausführungsform ist die Antriebssteuerung ausgelegt, um eine erste Geschwindigkeit vorzusehen, welche im Verknotungsbereich eingehalten wird.

In einer besonders bevorzugten Ausführungsform ist die Antriebssteuerung ausgelegt, um eine zweite Geschwindigkeit des Mitnehmers vorzusehen, welche in einem Umschlagsbereich eingehalten wird.

Der Umschlagsbereich kann im Sinne der vorliegenden Erfindung als ein Bereich verstanden werden, in dem eine oder mehrere vom Mitnehmer mitgeführte(n) Fadenstrecke(n) über einen Bahnspeisungsbereich gelegt werden, sodass sie sich um eine entsprechende mit einem Rückholfaden zu versehende Bahnstrecke eines bandförmigen Substrats legen. Analog kann im Sinne der vorliegenden Erfindung der Verknotungsbereich angesehen werden als ein weiterer Bereich, in dem mindestens zwei Fadenstrecken des Fadenendes dergestalt an das Knoter-Aggregat geführt werden, dass das Knoter-Aggregat diese beiden miteinander verknotet.

Im Betrieb würde dies dann bedeuten, dass im Umschlagsbereich ein Auge, eine Schlaufe, oder eine Schlinge eines Rückholfadenendes gebildet wird und im Verknotungsbereich die entstehenden freien Fadenenden verknüpft werden.

Für die Ausführung der vorliegenden Erfindung geeignete Knoter-Aggregate sind bekannt. In ihrer einfachsten Ausführungsform umfassen die Knoter-Aggregate, welche für die erfindungsgemässe Vorrichtung geeignet sind, mindestens einen Verknotungsdornen. Der Verknotungsdorn ist innerhalb eines Knotenrings drehbar gelagert und angetrieben. In der Regel rotiert der Verknotungsdornen in eine Rotationsrichtung, welche umgekehrt ist zur Rotationsrichtung des Mitnehmers auf der Umlaufbahn ist. Der Verknotungsdornen greift in die vom Mitnehmer herangeführten Fadenstrecken und vollführt in der Zeit eine Rotation, in der der Mitnehmer in dem Verknotungsbereich unterwegs ist. Durch die Rotation des Verknotungsdorns entsteht dann ein Knoten.

In einer besonderen Ausführungsform weist das Knoter-Aggregat eine Blende auf, welche geeignet ist, die beiden Fadenstrecken in den Wirkbereich des Knoter-Aggregats zu führen, wenn sie vom radial vorbeifahrenden Mitnehmer in den Wirkbereich des Knoter-Aggregats geführt werden.

In einer besonderen Ausführungsform ist die Vorrichtung ausgelegt, einen Knoten zu bilden, also ein Fadenende zu verknoten, während einer Umdrehung des Mitnehmers auf der Umlaufbahn.

In einer besonderen Ausführungsform ist der Mitnehmer ausgerichtet, um auf einer kreisförmigen Umlaufbahn radial geführt zu werden. In einer alternativen oder ergänzenden Ausführungsform ist der Mitnehmer mit der Umlaufbahn starr verbunden und die Umlaufbahn ist ausgelegt, um radial geführt zu werden.

In einer besonderen Ausführungsform umfasst die Vorrichtung weiter eine Fadenspeisung zur kontinuierlichen Zufuhr eines Fadenendes an den Mitnehmer.

In einer besonders bevorzugten Ausführungsform umfasst die Fadenspeisung eine Fadenspule mit einem aufgewickelten Faden, sowie insbesondere mindestens eine Abspulrolle, über welche der Faden mit dem Mitnehmer verbunden wird. In der Bewegung des Mitnehmers wird der Faden von der Fadenspule abgespult und einmal um die gesamte Vorrichtung geführt, sodass sich im Wesentlichen eine Schlaufe aus zwei Fadenstücken bildet.

Im Sinne der vorliegenden Erfindung kann unter einem Fadenende stets das im Hinblick auf die Gesamtlänge des Fadens auf der Spule betrachtet bearbeitete Endstück betrachtet werden.

Die Fadenstücke, wie sie im Sinne der vorliegenden Erfindung verstanden werden können, bilden die beiden Schenkel einer im Wesentlichen durch eine Umschlagsbewegung des Mitnehmers geformten Schlaufe, wobei ein Auge gebildet wird, in dem sich später das bandförmige Substrat befinden wird. Diese Schlaufe wird üblicherweise quer zur Längsrichtung des bandförmigen Substrats um dasselbe gelegt, sodass ein Ziehen am geknüpften Ende der Schlaufe quer zur Längsrichtung des bandförmigen Substrats Kraft ausübt.

Im Sinne der vorliegenden Erfindung kann als die dem Knoten entgegengesetzte Seite der Schlaufe auch das distale Ende des Rückholfadens bezeichnet werden, während der Knoten am proximalen Ende des Rückziehfadens zu liegen kommt. Dies entspräche der üblichen Terminologie bei Betrachtung eines Tampons, wobei das distale Ende in der Regel von einem konisch zugespitzten geformten Tamponkopf gebildet wird.

In einer besonderen Ausführungsform ist das Knoter-Aggregat ausgestaltet, um einen Knoten zu bilden indem der Verknotungsdorn entgegen der Rotationsrichtung des Mitnehmers um eine Rotationsachse rotiert. Insbesondere rotiert der Verknotungsdorn mit mehr als einer ganzen Umdrehung um seine Rotationsachse bei der Bildung des Knotens. Bevorzugt ist der Verknotungsdornen ausgelegt um zwischen 1 und 1.8 Umdrehungen beim Bilden des Knotens zu vollführen, insbesondere zwischen 1.1 und 1.6 Umdrehungen, ganz besonders bevorzugt ca. 1.5 Umdrehungen.

In einer besonderen Ausführungsform ist der Verknotungsdornen dergestalt angetrieben, dass er mehr als eine ganze Umdrehung entgegen der Rotationsrichtung des Mitnehmers vollführt, in dem Zeitraum, in dem sich der Mitnehmer durch den Verknotungsbereich bewegt. Dies kann im Betrieb bedeuten, dass der Verknotungsdornen zwischen 1 und 2 Umdrehungen vollführt, während der Mitnehmer einen Verknotungsbereich umläuft und wieder in eine Ausgangslage zurückkehrt, insbesondere durch Vollendung der angebrochenen Umdrehung, während der Mitnehmer den Umschlagsbereich abfährt.

In einer besonderen Ausführungsform umfasst die Vorrichtung weiter ein Schneidelement zum Abtrennen der geknoteten Schlaufe des Fadenendes. Besonders bevorzugt wirkt das Schneidelement an dem Fadenstück, welches am proximalen Ende der Schlaufe noch mit der Spule verbunden ist. Besonders bevorzugt ist das Schneidelement so angebracht, dass das Schneiden durch die Bewegung des Mitnehmers durchgeführt wird, d.h. der Mitnehmer das zu schneidenende Fadenstück am Schneidelement so vorbeiführt, dass dieses geschnitten wird. In seiner einfachsten Ausführungsform kann das Schneidelement eine in Wegrichtung des Mitnehmers angebrachte Klinge sein, durch welche der Mitnehmer den Faden gegen einen Widerstand führt, so dass der Faden von dem Schneidelement durchtrennt wird.

In einer besonderen Ausführungsform ist das Schneidelement gekrümmt. In einer besonderen Ausführungsform bildet die Schnittstelle des Schneidelements nun das neue Endstück, über welches der Mitnehmer weiteren Faden in der weiteren Umlaufbahn abspult und es entsteht ein neues Fadenende, um einen neuen Zyklus auszulösen.

In einer besonderen Ausführungsform ist die Umlaufbahn im Wesentlichen kreisförmig. Besonders bevorzugt ist die Umlaufbahn kreisförmig. Dadurch, dass der Faden mit einer kreisförmigen Bewegung des Mitnehmers abgespult wird, kann die Konstruktion insgesamt kompakter ausgestaltet werden und eine Zugänglichkeit der einzelnen Komponente verbessert werden. Zudem ist die Abnutzung der Komponenten geringer.

In dieser Ausführungsform können der Verknotungsbereich, der Beschleunigungsbereich, der Umschlagsbereich, der Bremsbereich als Winkel der kreisförmigen Umlaufbahn definiert sein. Die Winkel können der genannten Reihenfolge aufeinander folgen und sich überlappen. In einer weiteren besonderen Ausführungsform ist die Vorrichtung so ausgestaltet, dass der Mitnehmer einen Zyklus mit einer Umdrehung der Umlaufbahn um ihre Rotationsachse abschliesst, insbesondere um den Mittelpunkt einer kreisförmigen Umlaufbahn.

In einer besonderen Ausführungsform umfasst der Zyklus zwei Geschwindigkeiten in denen der Mitnehmer auf der kreisförmigen Umlaufbahn geführt wird. Eine erste Geschwindigkeit im Verknotungsbereich ist tiefer ausgelegt als eine zweite Geschwindigkeit im Umschlagsbereich. Besonderes bevorzugt ist die erste und die zweite Geschwindigkeit an die Umdrehungsgeschwindigkeit des Verknotungsdorns angepasst. Die erste Geschwindigkeit ist so bemessen, dass der Verknotungsdorn eine zwischen 1 und 2-fache Umdrehung um eine Rotationsachse zu vollführen vermag. Die zweite Geschwindigkeit ist so bemessen, dass der Verknotungsdorn die restliche Umdrehung, d.h. die Differenz zwischen einer Angefangenen und Beendeten zweiten Umdrehung zu vollführen vermag. So kann zum Beispiel sichergestellt werden, dass der Verknotungsdorn wieder im Ausgangszustand ist, und bereit ist, wenn der Mitnehmer erneut in den Verknotungsbereich eintritt. Alternativ kann der Verknotungsdorn auch mehr als die restliche Umdrehung, d.h. die Differenz zwischen einer Angefangenen und Beendeten zweiten Umdrehung vollführen, nämlich zusätzlich noch N ganze Umdrehungen vollführen. Wobei eine ganze Umdrehung eine vollständige Rotation um die Rotationsachse darstellt, und einen Winkel von 360° definiert.

In einer besonderen Ausführungsform umfasst die erfindungsgemässe Vorrichtung einen Bahneinlass zur Durchfuhr eines bandförmigen Substrats durch den Wirkbereich des Mitnehmers.

In einer besonderen Ausführungsform ist dieser Bahneinlass eine Ausnehmung, durch welche das bandförmige Substrat in den Wirkbereich des Mitnehmers förderbar ist. Im Sinne der vorliegenden Erfindung ist der Wirkbereich des Mitnehmers durch seine Radialbewegung auf der Umlaufbahn definiert. Mit anderen Worten ist der Wirkbereich des Mitnehmers gegeben, sobald ein Objekt sich innerhalb der Umlaufbahn des Mitnehmers befindet. In der Praxis kann dies bedeuten, dass der Mitnehmer ein Fadenende abspult und auf der Umlaufbahn so mitführt, dass er es um ein im Bahneinlass befindliches bandförmiges Substrat legt.

In einer besonderen Ausführungsform ist dementsprechend der Bahneinlass lateral zum Knoter-Aggregat ausgebildet. Besonders bevorzugt wird das Fadenende um das bandförmige Substrat gelegt bei einem Umlauf des Mitnehmers auf der Umlaufbahn mit der zweiten Geschwindigkeit.

Die Vorrichtung umfasst eine Haltevorrichtung, um eine durch den Umlauf des Mitnehmers auf der Umlaufbahn gebildete Schlaufe zu halten. Besonders bevorzugt ist die Haltevorrichtung lateral zum Knoter-Aggregat ausgebildet. Ganz besonders bevorzugt ist die Haltevorrichtung lateral zum Bahneinlass ausgebildet, sodass die Schlaufe dergestalt gehalten wird, dass ein bandförmiges Substrat innerhalb der Schenkel der Schlaufe zu liegen kommt.

Die Haltevorrichtung ist als Klinke ausgebildet. Die Klinke ist ausgelegt, eine Rückstellkraft auf ein Schlaufenende des zu verknotenden Fadenendes auszuüben. Insbesondere ist die Klinke ausgebildet um eine lösbare Rückstellkraft auf ein Schlaufenende des zu verknotenden Fadenendes auszuüben.

In einer besonderen Ausführungsform ist die Haltevorrichtung angetrieben. Dies kann z.B. dadurch erreicht werden, dass die Haltevorrichtung mit einem aktivierbaren pneumatischen Zylinder versehen ist.

In einer ergänzenden Ausführungsform ist die Haltevorrichtung mit einer Rückstellkraft ausübenden Komponente ausgestattet, sodass die Haltevorrichtung eine Spannung auf der gehaltenen Schlaufe im Hinblick auf den Mitnehmer und das Knoter-Aggregat aufrechterhält. In der Variante, in der die Haltevorrichtung als Klinke ausgebildet ist, kann sie zudem mit einem mechanischen Endanschlag ausgestattet sein, welcher die ausgefahrene Klinke arretiert. Beim Lösen der Klinke kann sie eine Schlaufe lösen und in einen gelösten Zustand übergehen. Bevorzugt ist die Klinke dann so ausgestattet, dass sie automatisch in ihre Ursprungslage versetzbar ist.

In einer besonderen Ausführungsform umfasst die Klinke einen pneumatischen Zylinder.

In einer alternativen oder ergänzten Ausführungsform umfasst die Klinke eine Feder.

In einer besonderen Ausführungsform ist die Haltevorrichtung im Hinblick auf einen Bahneinlass verstellbar ausgestaltet, sodass verschiedene Schlaufenlängen akkommodierbar sind. So können z.B. je nach gewählter Distanz der Haltevorrichtung zum Bahneinlass, resp. zu einem Knoter-Aggregat entsprechend längere oder kürzere Fadenstrecken für die Bildung der Schlaufe verwendet werden.

In einer besonderen Ausführungsform umfasst die Haltevorrichtung mindestens zwei Haltezähne. Die Haltezähne können so ausgestaltet sein, dass jeweils ein Ende einer Fadenstrecke von einem Haltezahn gehalten wird, sodass die Haltevorrichtung ein entsprechendes Auge einer Schlaufe aufhält, in dem z.B. ein Bahneinlass geführt sein kann, um das bandförmige Substrat hindurchzuführen.

In einer besonderen Ausführungsform ist die Klinke so ausgestaltet, dass sie eine Haltekraft auf die Schlaufe ausübt, solange diese einen Gegenzug aufweist. Im Betrieb kann dies bedeuten, dass, sobald ein Knoten geknüpft wurde und das Fadenende abgetrennt wurde, ein Gegenzug nicht mehr besteht, da auf die Schlaufe nicht länger vom Mitnehmer eine Zugkraft ausgeübt wird. Die Klinke kann so ausgestaltet sein, dass durch Mangel eines Gegenzugs die Schlaufe entlassen wird. Dieses Entlassen der Schlaufe kann dazu führen, dass sie von einem geförderten bandförmigen Substrat, um den sie gelegt ist, mitgezogen wird.

In einer besonderen Ausführungsform umfasst die Vorrichtung ein Führungsblech zum Führen des bandförmigen Substrats durch den Wirkbereich des Mitnehmers. Dieses Führungsblech kann z.B. rechtwinkelig zum Bahneinlass angeordnet sein.

In einer besonderen Ausführungsform ist das Führungsblech so ausgelegt, dass nach Lösen einer Haltevorrichtung eine gebildete Schlaufe zunächst auf das Führungsblech umgelegt wird und durch die Förderung des bandförmigen Substrats anschliessend an den nächsten Bearbeitungsschritt mitsamt der entsprechenden Bahnsubstratstrecke weitergegeben wird.

In einer besonderen Ausführungsform weist das Führungsblech in Längsrichtung des bandförmigen Substrats eine verjüngte Stirnseite auf.

In einer besonderen Ausführungsform ist die Breite des Führungsblechs an die Länge des zu erzielenden Rückholfadens ausgerichtet.

In einer besonderen Ausführungsform umfasst die erfindungsgemässe Vorrichtung weiter eine Knotenkontrolle zur Überprüfung des vom Knoter-Aggregat geknüpften Knotens. Insbesondere kann die Knotenkontrolle eine optische, mechanische und/oder elektrische Knotenkontrolle umfassen. So kann eine optische Knotenkontrolle z.B. erreicht werden, indem eine Kamera in den Knoter-Bereich geführt ist und die Knoten optisch prüft. Eine mechanische Knotenkontrolle kann vorgesehen sein, welche ein Weiterfördern des geknüpften Rückholfadens verhindert, wenn kein Knopf besteht.

In einer besonderen Ausführungsform ist die Umlaufbahn antreibbar. Besonders bevorzugt weist die Umlaufbahn eine Verzahnung auf, durch welche sie mittels eines Zahnriemens antreibbar ist. Besonders bevorzugt ist sie gleichmässig beschleunigbar und vollführt eine Rotation um eine Rotationsachse. In dieser Ausführungsform können die Mitnehmer starr mit der Umlaufbahn verbunden sein, indem sie mit dieser vernietet und/oder verschraubt sind.

Besonders bevorzugt sind die Mitnehmer austauschbar ausgestaltet und können lösbar an der antreibbaren Umlaufbahn befestigt werden. In dieser Ausführungsform kann ein Zahnriemen mit einem Riemenantrieb verbunden sein, welcher über eine Antriebssteuerung in der Lage ist, die Umlaufbahn erfindungsgemäss bereichssegmentiert zu beschleunigen, resp. zu bremsen. Dies kann dazu führen, dass die Umlaufbahn einen Radius beschreibt, in dem verschiedene Geschwindigkeiten des Mitnehmers vorgesehen sind.

In einer besonderen Ausführungsform ist die Antriebssteuerung so ausgestaltet, dass ein stufenlos beschleunigbarer Umlaufantrieb möglich ist.

In einer besonderen Ausführungsform umfasst das Knoter-Aggregat mindestens einen Verknotungsdornen, der parallel zur Rotationsrichtung der Umlaufbahn rotierbar angetrieben ist. Mit der vorliegenden Erfindung ist eine Vorrichtung zum Verknoten eines Fadenendes, sodass eine Schlaufe gebildet wird, bereitgestellt, welche einfach in der Wartung ist und ausgelegt ist, um in einer hohen Frequenz bandförmige Substrate mit den entsprechenden Schlaufen zu versorgen, sodass diese zu Rückholfäden von Tampons gebildet werden können. Die erfindungsgemässe Vorrichtung vollführt dies in einer kontinuierlich arbeitenden Weise und ist somit an die Anforderungen moderner kontinuierlicher Herstellungsprozesse angepasst.

Für einen Fachmann versteht es sich von selbst, dass sämtliche genannte Ausführungsformen in einer erfindungsgemässen Ausgestaltung miteinander kombiniert vorkommen können, sofern sich diese nicht gegenseitig ausschliessen.

Ein weiterer Aspekt der vorliegenden Erfindung ist ein Verfahren zur Verknotung eines Fadenendes, insbesondere von zwei Fadenstrecken eines Fadenendes, sodass eine Schlaufe entsteht, welche einen Rückholfaden für Tampons bildet.

In einer besonderen Ausführungsform wird dieses Verfahren mit einer erfindungsgemässen, wie oben geschilderten Vorrichtung ausgeführt. Entsprechend sind die funktionalen Merkmale, die oben in Bezug auf die Vorrichtung geschildert wurden, gleichzeitig mögliche Verfahrensmerkmale des erfindungsgemässen Verfahrens.

Das erfindungsgemässe Verfahren umfasst den Schritt des Führens eines Fadenendes eines Fadens mittels eines auf einer Umlaufbahn bewegbaren Mitnehmers entlang der Umlaufbahn um ein Knoter-Aggregat und ein Umschlagselement, sodass zwischen Knoter-Aggregat und Umschlagselement eine Schleife aus zwei Fadenstrecken des Fadenendes gebildet wird.

Das erfindungsgemässe Verfahren umfasst weiter den Schritt des Verknotens der zwei Fadenstrecken durch ein Knoter-Aggregat und Lösen der Schlaufe am Umschlagselement. Im erfindungsgemässen Verfahren weist die Umlaufbahn einen Verknotungsbereich auf, der mit einer ersten Geschwindigkeit vom Mitnehmer durchfahren wird, sowie einen Überschlagsbereich auf, der vom Mitnehmer mit einer zweiten Geschwindigkeit durchfahren wird. Zu diesem Zweck kann zwischen Verknotungsbereich und Überschlagsbereich an den Übergängen je ein Beschleunigungsbereich, in dem der Mitnehmer auf eine zweite Geschwindigkeit beschleunigt wird, und ein Bremsbereich auf, in dem der Mitnehmer auf eine erste Geschwindigkeit gebremst wird, vorgesehen sein.

In einer besonderen Ausführungsform ist das Verhältnis der ersten Geschwindigkeit zur zweiten Geschwindigkeit zwischen zwei zu eins und sechs zu eins.

Im Betrieb würde beispielsweise durch einen Mitnehmer ein Fadenende von einer Fadenspule auf der Umlaufbahn geführt. Diese Umlaufbahn führt den Faden am Umschlagselement vorbei, sodass zwischen Umschlagselement und Mitnehmer eine Spannung entsteht. Weiter entsteht so ein Auge einer Schleife. Auf seinem weiteren Weg führt der Mitnehmer nun zwei Fadenstrecken zum Knoter-Aggregat, und ein dieses verknotet die beiden Fadenenden.

In einer besonderen Ausführungsform des erfindungsgemässen Verfahrens geschieht eine Zufuhr eines Fadenendes von einem Faden auf einer Spule auf den über die Umlaufbahn bewegbaren Mitnehmer über eine Rückstellkraft. Dies kann dazu führen, dass eine stetige Spannung auf den Faden aufrechterhalten wird, ungeachtet der Bahn, die der Mitnehmer auf der Umlaufbahn vollführt. Zu beachten ist, dass, obschon der Mitnehmer eine kreisförmige Umlaufbahn beschreiten kann, der Faden direkt von der Fadenspule oder einer allfällig dazwischengeschalteten Ablenkrolle geführt sein kann.

Eine Rückstellkraft kann auf den Faden wirken, indem z.B. ein gefederter Hebel oder eine angetriebene Zwischenspule zwischen dem Mitnehmer und der Fadenspule angeordnet ist. In einer besonderen Ausführungsform sind diese Elemente ausserhalb des Radius der Umlaufbahn angeordnet.

In einer besonderen Ausführungsform umfasst das Lösen der Schlaufe ein Abschneiden des Fadenendes von dem Faden. Dieses Abschneiden kann im Wesentlichen zeitgleich mit dem Verknoten der Fadenstrecken stattfinden. Insbesondere findet dieses Abschneiden kurz nach dem Verknoten der Fadenstrecken statt.

In einer besonders bevorzugten Ausführungsform läutet das Abschneiden des Fadenendes ebenso das Lösen der Schlaufe ein, indem diese durch einen fehlenden Gegenzug daran gehindert wird, eine Rückstellkraft auf die verknotete Schlaufe auszuüben und diese somit gelöst wird.

In einer besonderen Ausführungsform ist das Verhältnis der ersten Geschwindigkeit zur zweiten Geschwindigkeit zwischen eins zu zwei und eins zu sechs. Mit anderen Worten findet die Bewegung des Mitnehmers im Bereich der ersten Geschwindigkeit mit einer Geschwindigkeit statt, die im Wesentlichen zwischenhalb so hoch bis sechs mal kleiner ist, als die Geschwindigkeit in einem zweiten Bereich.

In einer weiteren besonderen Ausführungsform handelt es sich bei der ersten Geschwindigkeit um die Geschwindigkeit, die der Mitnehmer aufweist, wenn er einen Verknotungsbereich durchläuft, das heisst einen Bereich durchläuft in dem die Fadenstrecken beim Knoter-Aggregat sind und das Verknoten der Fadenstrecken durch den Verknotungsdornen stattfindet.

In einer besonders bevorzugten Ausführungsform sind der Verknotungsbereich, der Umschlagsbereich, sowie Beschleunigungs-, resp. der Bremsbereich als Winkel einer kreisförmigen Umlaufbahn definiert. In einer besonderen Ausführungsform umfasst der Verknotungsbereich einen Bereich von zwischen 5 und 45 ° der Umlaufbahn, bevorzugt zwischen 10 und 30° der Umlaufbahn, weiter bevorzugt 15° der Umlaufbahn. Entsprechend bildet der Rest einer vollständigen Umlaufbahn, also die übrigen zwischen 355 und 315 °, insbesondere zwischen 350 und 330 °, weiter insbesondere 345° den Umschlagsbereich.

Besonders bevorzugt weist die Umlaufbahn einen Beschleunigungsbereich und einen Bremsbereich auf, in denen die entsprechenden Geschwindigkeiten aufgenommen werden. Besonders bevorzugt ist der Beschleunigungsbereich und der Bremsbereich im Umschlagsbereich angeordnet.

In diesen Bereichen wird der Mitnehmer auf die entsprechende Geschwindigkeit beschleunigt oder gebremst, die er im Umschlagsbereich fährt, respektive im Verknotungsbereich. Besonders bevorzugt geschieht das Beschleunigen und das Bremsen des Mitnehmers über einen Antrieb und ist von einer Antriebssteuerung gesteuert. In diesem Beispiel könnte eine Antriebssteuerung ausgelegt sein, um intermittierend eine Bewegung des Mitnehmers zu steuern, welche von einer ersten Geschwindigkeit in eine zweite übergeht und wieder zurück, und dies in einem Takt, so dass stets dieselben Winkelbereiche einer Umlaufbahn, auf dem der Mitnehmer sich bewegt, für die entsprechende Beschleunigung oder Bremsung verwendet werden.

In einer besonderen Ausführungsform des erfindungsgemässen Verfahrens kann eine Position Startposition P₀ angesehen werden, zum Beispiel eine Position in der der Mitnehmer einen Winkel zum Mittelpunkt einer kreisförmigen Umlaufbahn einschliesst, der 0° oder im Wesentlichen 0° beträgt. Im Kontext der vorliegenden Erfindung ist im Wesentlichen 0°, resp. im Wesentlichen X° als eine Abweichung von höchstens 1° zu verstehen.

In einer besonderen Ausführungsform schliesst der Verknotungsbereich einen Winkel von zwischen 0 und 45° ein, insbesondere von zwischen 0 und 30° ein, insbesondere von 15° ein.

In einer weiteren besonderen Ausführungsform schliesst ein Beschleunigungsbereich an einen Verknotungsbereich an. In einer weiteren besonderen Ausführungsform ist der Verknotungsbereich in einem Winkel von zwischen 0° und 15° und direkt im Anschluss beginnt der Beschleunigungsbereich.

Das Lösen der Schlaufe am Umschlagselement sieht ein Lösen einer rückstellbaren Klinke vor. Die Klinke ist ausgestaltet, um eine lösbare Rückstellkraft in Richtung des Mitnehmers und/oder Knoter-Aggregats auszuüben. Die Klinke ist bevorzugt so ausgestaltet, dass sie auslösbar ist und automatisch in ihren ursprünglichen Zustand rückführbar ist. Dies kann z.B. mittels eines pneumatischen Zylinders geschehen oder auch mittels einer mit einer eingestellten Rückstellkraft versehenen Feder. Ebenfalls denkbar wäre es, mit einer magnetischen Steuerung die Klinke jeweils von einer gelösten in eine Rückstellkraft ausübende Position zu überführen.

In einer besonderen Ausführungsform umfasst das erfindungsgemässe Verfahren weiter die Schritte des Bereitstellens einer Vorrichtung zur Verknotung eines Fadenendes, wie oben geschildert, und die Durchfuhr eines bandförmigen Substrats durch den Wirkbereich des Mitnehmers, sodass das Fadenende um das bandförmige Substrat gelegt wird. Insbesondere wird das Fadenende so um das bandförmige Substrat gelegt, dass bei einem Umlauf des Mitnehmers auf der Umlaufbahn das Fadenende um das bandförmige Substrat gelegt wird, wobei die Durchfuhr des bandförmigen Substrats im Wesentlichen normal zu einer Umlaufbahn eines Mitnehmers angeordnet ist.

Im Betrieb kann eine erfindungsgemässe Vorrichtung dergestalt in eine Anlage zur Herstellung von Tampons eingebaut werden, indem sie senkrecht zur Rotationsachse der Umlaufbahn, resp. des Mitnehmers mit der Vorrichtung in Wirkverbindung gebracht wird. Dazu kann eine entsprechende Zufuhrblende vorgesehen sein, welche ein bandförmiges Substrat z.B. in eine wie oben geschilderte Bahnspeisung einzuführen vermag. Die Bahnspeisung ist so ausgelegt, dass eine gebildete Schlaufe durch die Bewegung des Mitnehmers um die Bahnspeisung herum angeordnet ist, sodass ein bandförmiges Substrat im Inneren dieser Bahnspeisung senkrecht, oder mindestens im Wesentlichen normal zur Längsausrichtung des Fadens angeordnet ist. Wird die Schlaufe gebildet und findet ein Lösen statt, so fällt die Schlaufe auf das bandförmige Substrat.

In einer besonderen Ausführungsform ist das bandförmige Substrat über ein Führungsblech geführt, auf das zunächst die Schlaufe fällt und im weiteren Verlauf durch eine sich verjüngende Stirnseite auf das bandförmige Substrat gelegt wird, ohne dass dabei das bandförmige Substrat in seiner Förderbahn beeinträchtigt wird.

Entsprechend ist es ein weiterer Aspekt der vorliegenden Erfindung, eine Vorrichtung zur Herstellung von Tampons mit einem proximalen Rückholfaden bereitzustellen. Der Rückholfaden umfasst zwei miteinander verknüpfte Fadenenden. Die Vorrichtung umfasst eine Vorrichtung zur Verknotung zweier Fadenenden, wie eingangs geschildert.

Sie umfasst weiter eine Fördervorrichtung zur Förderung von bandförmigem Substrat in einen Wirkbereich der Vorrichtung zur Verknotung der beiden Fadenenden. Insbesondere ist die Fördervorrichtung im Wesentlichen normal zu einer Umlaufbahn eines Mitnehmers zum Zuführen von zwei Fadenstrecken an ein Knoter-Aggregat angeordnet.

Ein weiterer Aspekt und/oder besondere Ausführungsform der vorliegenden Erfindung betrifft eine Vorrichtung zur Verknotung eines Fadenendes so dass eine Schlaufe um ein bandförmiges Substrat gebildet wird, insbesondere eines Fadenendes eines Rückholfadens für Tampons. Die Vorrichtung kann zudem alle nicht in Widerspruch mit den folgenden Merkmalen stehenden Merkmale der oben genannten Vorrichtung umfassen.

Diese Vorrichtung umfasst ein Knoter-Aggregat zur Verknotung von zwei Fadenstrecken des Fadenendes.

Sie umfasst weiter einen Mitnehmer zum Zuführen der Fadenstrecken an das Knoter-Aggregat, wobei der Mitnehmer entlang einer Umlaufbahn um einen Bahneinlass zur Durchfuhr eines bandförmigen Substrats rotierbar gelagert ist, insbesondere entlang einer kreisförmigen Umlaufbahn rotierbar gelagert ist. Die Vorrichtung umfasst zudem einen im Wesentlichen normal zum Bahneinlass angeordneten Abnehmer, der ausgebildet ist, um eine Fadenstrecke vom Mitnehmer aufzunehmen, und auf das bandförmige Substrat abzulegen. Im Kontext der vorliegenden Erfindung sei im Wesentlichem normal zu verstehen, als hinsichtlich der Ebene des bandförmigen Substrats innerhalb eines Winkelbereichs von zwischen 85 ° und 95 ° angeordnet.

Durch einen dergestalt angeordneten Abnehmer kann der Faden sanft auf das bandförmige Substrat, zum Beispiel ein Watteband, abgelegt werden. Insgesamt wird die Ablagebewegung beruhigt, was es ermöglicht die Prozessgeschwindigkeit zu erhöhen und die Zuverlässigkeit zu verbessern. Die Vorrichtung läuft trotz der hohen Geschwindigkeit insgesamt ruhiger. Durch die erhöhte Zuverlässigkeit können auch die Wartungsintervalle verkürzt und die Störungen gesenkt werden, was insgesamt auch eine bessere Auslastung der Vorrichtung gestattet.

In einer besonderen Ausführungsform ist der Abnehmer im Wesentlichen aus Metall. Dadurch kann die Haltbarkeit erhöht und der Verschleiss gesenkt werden. Besonders bevorzugt ist der Abnehmer aus Edelstahl. Weiter bevorzugt ist der Abnehmer mindestens in seinen beweglichen Teilen in einer Leichtbauweise gefertigt, d.h. wo immer möglich wird Material gespart, zum Beispiel indem Ausnehmungen in einem Abnehmerhebel vorgesehen sind, die zu Material- und somit auch Gewichtseinsparungen führen können.

Alternativ ist der Abnehmer, insbesondere mindestens ein Abnehmerhaken zur Aufnahme einer Fadenstrecke aus einem keramischen Material.

In einer besonderen Ausführungsform umfasst die Vorrichtung ein Führungsblech zum Führen des bandförmigen Substrats durch den Wirkbereich des Mitnehmers. In einer weiteren besonderen Ausführungsform umfasst die Vorrichtung ein Anschlagsblech. Dieses Anschlagsblech kann zwischen dem Führungsblech und dem Abnehmer positioniert sein, so dass das bandförmige Substrat beim Ablegen der Fadenstrecke durch den Abnehmer nicht von diesem berührt wird. Dies kann die Laufruhe der Vorrichtung erhöhen.

In einer besonderen Ausführungsform umfasst der Abnehmer eine Abnehmerfeder, die auf einen zur Aufnahme einer Fadenstrecke ausgebildeten Abnehmerhaken eine Rückstellkraft ausübt. Die Rückstellkraft ist bevorzugt so eingestellt, dass sie durch das Verknoten zweier Fadenstrecken des Fadenendes durch das Knoter-Aggregat überwunden wird. Im Betrieb würde dann zum Beispiel das Verknoten zu einem Absenken des Abnehmerhebels gegen die Rückstellkraft der Abnehmerfeder führen, wodurch die so gebildete Schlaufe auf das Substratband sanft abgelegt werden kann. Sobald die Fadenstrecke aus dem Abnehmerhaken entlassen ist, kann die Rückstellkraft auf den Abnehmerhaken dergestalt einwirken, dass dieser wieder in seine ursprüngliche Position zurückschnellt, bereit für die Aufnahme einer nächsten Fadenstrecke eines folgenden Fadenendes.

Durch das im Wesentlichen sanfte Abgleiten einer Schlaufe auf das bandförmige Substrat kann die Laufruhe der Vorrichtung erhöht, und eine weitere Erhöhung der Prozessgeschwindigkeit ermöglicht werden.

In einer besonderen Ausführungsform umfasst der Abnehmer, der ausgebildet ist, um eine Fadenstrecke vom Mitnehmer auf das bandförmige Substrat abzulegen ein Federelement, welches auf den Abnehmer eine Rückstellkraft ausübt. Das Federelement kann nach einem Ablegen einer Fadenstrecke ein Rückführen des Abnehmers in einen angewinkelten Zustand erleichtern.

In einer besonderen Ausführungsform weist das Knoter-Aggregat eine Blende auf, welche geeignet ist, die beiden Fadenstrecken in den Wirkbereich des Knoter-Aggregats zu führen, wenn sie vom radial vorbeifahrenden Mitnehmer in den Wirkbereich des Knoter - Aggregats geführt werden.

In einer besonderen Ausführungsform weist das Knoter-Aggregat eine Knoter-Öhse auf, welche geeignet ist, mindestens eine Fadenstrecke in den Wirkbereich des Knoter-Aggregats zu führen, wenn sie vom radial vorbeifahrenden Mitnehmer in den Wirkbereich des Knoter-Aggregats geführt werden. Bevorzugt weist die Knoter-Öhse zu diesem Zweck eine Führungsgeometrie auf, die entgegen der Rotationrichtung des Mitnehmers fluchtend ausgestaltet ist.

Die erfindungsgemässe Vorrichtung zur Herstellung von Tampons weist eine Fördervorrichtung zur Beförderung des bandförmigen Substrats auf, die dergestalt in den Wirkbereich der Vorrichtung zur Verknotung zweier Fadenenden fördert, dass bei einer Umlaufbewegung eines Mitnehmers entlang einer Umlaufbahn das Fadenende im Wesentlichen quer zur Längsachse des bandförmigen Substrats um dieses gelegt wird.

Im Folgenden wird nun die vorliegende Erfindung anhand von Figuren und konkreter Ausführungsbeispiele näher erläutert, ohne auf diese beschränkt zu sein. Für einen Fachmann ergeben sich aus dem Studium dieser Beispiele und Figuren weitere vorteilhafte Ausführungsformen, die in einer erfindungsgemässen Lösung verwirklicht werden können.

Die Figuren sind schematisch, und zur einfacheren Nachverfolgung wurden die gleichen Teile mit den gleichen Bezugszeichen versehen.

### Fig urenbeschrieb

Anhand der nachfolgenden Figuren werden Ausführungsbeispiele der Erfindung beschrieben. Es zeigen:
- Fig. 1: schematisch den Aufbau einer erfindungsgemässen Vorrichtung;
- Fig. 2: schematisch eine Bereichsgliederung einer Umlaufbahn;
- Fig. 3: ein Beispiel einer erfindungsgemässen Vorrichtung;
- Fig. 4a: schematisch eine Klinke, wie sie in einer erfindungsgemässen Vorrichtung verwendet werden kann in einer gespannten Lage;
- Fig. 4b: die entsprechende Klinke der Fig. 4a in einer entspannten Lage;
- Fig. 5a: ein weiteres Beispiel einer erfindungsgemässen Vorrichtung;
- Fig. 5b: alternative Ausführungsform der Vorrichtung gemäss Fig. 5a
- Fig. 6: eine schematische Darstellung eines geeigneten Knoter-Aggregats;
- Fig. 7a: eine Ansicht eines Verknotungsdorns ohne Öhse;
- Fig. 7b: eine Ansicht eines Verknotungsdorns mit Öhse, und
- Fig. 8a: ein alternatives Beispiel einer erfindungsgemässen Vorrichtung;.
- Fig. 8b: schematischer Ausschnitt mit Detailansicht eines entspannten Abnehmers der Ausführungsform 8a;
- Fig. 8c: schematischer Ausschnitt mit Detailansicht eines gespannten Abnehmers der Ausführungsform 8a;
- Fig. 8d: schematischer Ausschnitt mit Detailansicht eines Knoter-Aggregats der Ausführungsform 8a, und
- Fig. 9: schematisch den Aufbau der Ausführungsform gemäss Fig. 8a.

### Ausführung der Erfindung

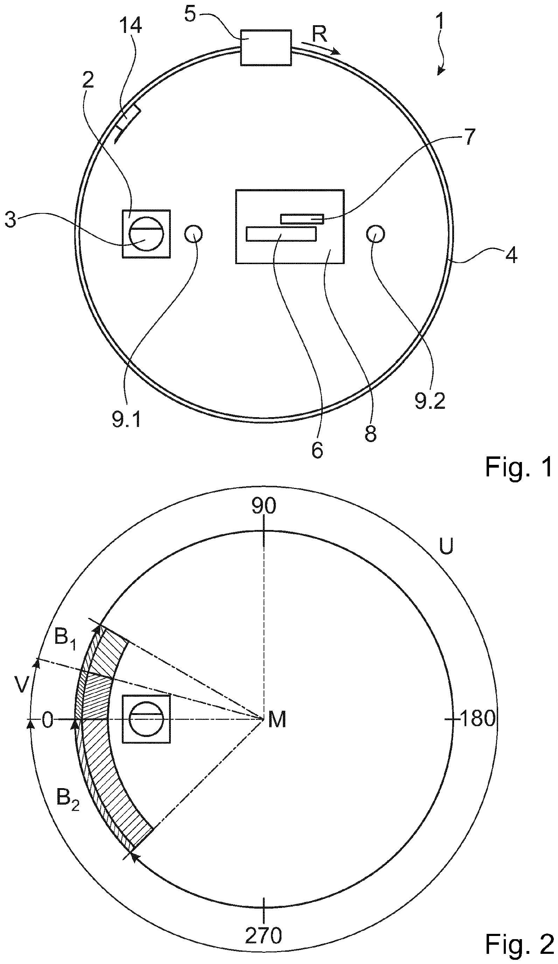

Die **Fig. 1** zeigt einen schematischen Aufbau einer erfindungsgemässen Vorrichtung 1 zur Verknotung eines Fadenendes. Ziel des Verknotens ist es, dass zwei lose Enden eines Fadens zunächst ein Auge bilden, und die losen Enden dergestalt verknotet werden, dass sie zusammen eine feste Schlaufe bilden. Im geschilderten Anwendungsverfahren ist die Schlaufe um ein bandförmiges Material geschlagen, so dass beide Schenkel der Schlaufe je auf einer Seite senkrecht zur Längsrichtung des Bandes zu liegen kommen. Wird das bandförmige Material aufgerollt, zum Beispiel zu einem Tampon für die Frauenhygiene, so kommt die Schlaufe im Inneren des Tampons zu liegen. Die verknoteten Fadenenden ragen aus dem Tampon heraus und der Knoten und die mit dem Knoten gebildete Schlaufe können als Rückholfaden dienen, zum Beispiel um den Tampons zu entnehmen.

Für die Vorrichtung 1 ist zum Zweck des Umschlagens um ein Bandmaterial und Verknotens der Fadenenden zunächst eine Umlaufbahn 4 vorgesehen. Im vorliegenden Beispiel ist die Umlaufbahn kreisförmig ausgebildet. Durch die kreisförmige Ausgestaltung der Umlaufbahn können vorteilhafte Effekte erzielt werden. Auf einer kreisform bewegte Objekte können mit einer konstanten Radialgeschwindigkeit gefahren werden. Lager und/oder Antriebsmittel erfahren im Wesentlichen eine gleichförmige Belastung, und können entsprechend so ausgestaltet sein, dass Unwucht-Effekte vermieden oder wenigstens minimiert werden. Ausserdem ist die Ansteuerung der einzelnen Elemente (siehe dazu später Synchronisation des Verknotungsdorns mit dem Fadenmitnehmer) vereinfacht. Beschleunigen und Bremsen der Elemente kann in Abstimmung auf Winkelsegmente erfolgen, so dass auch Feinjustierungen möglich sind - solche sind insbesondere dann sinnvoll, wenn eine erfindungsgemässe Vorrichtung an ein neues Fadenmaterial und/oder Bandmaterial angepasst wird. Hierzu kann der Fachmann abhängig von Fadenspannung, Elastizität und Reibungswiderstand die Ansteuerung die Bewegung der Elemente bei Bedarf anpassen.

Im vorliegenden Beispiel wird auf dieser Umlaufbahn 4 ein Mitnehmer 5 mit verschiedenen Geschwindigkeiten in eine Rotationsrichtung R bewegt. Der Einfachheit halber entspricht diese Rotationsrichtung R dem Uhrzeigersinn und wird im weiteren Verlauf der Anmeldung zur Erläuterung der Rotationsrichtung anderer Bauteile als Referenz genommen. Selbstverständlich kann eine erfindungsgemässe Vorrichtung auch spiegelverkehrt gestaltet sein, und die entsprechenden Rotationsrichtungen sich genau gegenläufig verhalten.

Der Mitnehmer 5 dient dazu, von einer Fadenspule (in Fig. 1 nicht gezeigt) einen Faden auf der Umlaufbahn 4 mitzuführen und diesen dabei um bestimmte Elemente der Vorrichtung 1 zu legen.

Auf seinem Weg legt der Mitnehmer 5 einen Faden insbesondere um einen ersten Stift 9.2, welcher im vorliegenden Beispiel als Umschlagselement dient. Der Stift 9.2 hält bei fortlaufender Bewegung um die Umlaufbahn ein Auge offen. Bei der Bewegung auf der Umlaufbahn 4 vollführt der Mitnehmer 5 insgesamt einen vollständigen Kreis und lässt den Faden, welcher z.B. von einer Fadenspule am gegenüberliegenden Rand des ersten Stifts 9.2 angeordnet sein kann, eine Schlaufe bilden zwischen Fadenspule und einem zweiten Stift 9.1. In der einfachsten Ausgestaltung ist dieser zweite Stift 9.1 als Dorn oder Schwert ausgestaltet, der von einer Rückwand, auf der die Elemente der Vorrichtung platziert werden und unter Umständen auch antreibbar sind in den Wirkungsraum des Mitnehmers 5 ragt. Im vorliegenden einfachsten Fall ist der erste Stift 9.1 ebenso ein Dorn, zum Beispiel ein sich in Richtung des Betrachters der Fig.1 hin verjüngender Dorn, von dem eine geknüpfte Fadenschlaufe einfach in die Richtung des Betrachters abgleiten kann, zum Beispiel in dem die Fadenschlaufe von einer normal zur Umlaufbahn 4 der Vorrichtung 1 verlaufenden Bandförderung mitgenommen wird. In besonderen und besonders vorteilhaften Ausgestaltungen kann der erste Stift 9.2 durch einen aktuierten Hebel, oder eine andere angetriebene Haltevorrichtung ersetzt sein, wie z.B. durch eine auslösbare Klinke.

Um zu verhindern, dass das vom als Dorn ausgebildeten ersten Stift ausgelöste Fadenende mit der Schlaufe zu stark auf das bandförmige Substrat aufschlägt, ist eine Ablenkfinne 7 vorgesehen, welche sich ebenfalls in den Wirkbereich und in das Innere der Schlaue erstreckt, diese beim Ablösen vom Dornen auffängt und sanft, such ein sich verjüngendes Ende in Bandlaufrichtung auf das bandförmige Material entlässt.

Durch einen Bahneinlass 8 gelangt bandförmiges Material in den Wirkungsbereich des Mitnehmers 5. In der einfachsten Ausgestaltung ist der Bahneinlass 8 eine Ausnehmung in der Rückwand der Vorrichtung. Ein Bandführungsblech 6 ragt in den Wirkbereich der Vorrichtung durch den Bahneinlass 8 und ist so angeordnet, dass ein bandförmiges Material über eine Antriebsvorrichtung, zum Beispiel Rollen (nicht gezeigt), in die Vorrichtung geführt ist. Auf diesem Bandführungsblech 6 wird bandförmiges Material in den Wirkbereich des Mitnehmers 5 gefördert. Hat der Mitnehmer den zweiten Führungsstift 9.1 passiert, so befindet sich das Bandführungsblech 6 und somit das bandförmige Substrat innerhalb eines durch zwei Schenkel des vom Mintnehmers 5 mitgeführten Fadens gebildeten Auges einer Schlaufe.

Im Verlauf dieser Bewegung durchfährt der Mitnehmer 5 einen Umschlagsbereich mit einer bestimmten Geschwindigkeit.

Am gegenüberliegenden Ende des ersten Stifts 9.1 befindet sich ein Knoter-Aggregat 3, welches innerhalb einer Knoter-Öhse 2 angeordnet ist.

Im vorliegenden Beispiel fährt der Mitnehmer nun mit einer Geschwindigkeit durch einen Verknotungsbereich, die von der Geschwindigkeit des Umschlagbereichs verschieden ist. So kann der Mitnehmer 5 mit einer Geschwindigkeit, die zwischen doppelt bis fünffach so hoch ist wie die Geschwindigkeit, als die, sie er im Verknotungsbereich fährt den Umschlagsbereich abfahren. Dieser Verknotungsbereich beginnt dort, wo die Wirkung des Knoter-Aggregats auf das Fadenende zu wirken beginnt, respektive dort, wo das Knoter-Aggregat die zwei Schenkel der Schlaufe verknüpft und einen Knoten bildet. Im vorliegenden Beispiel beginnt der Verknotungsbereich in etwa dort nachdem der Mitnehmer 5 den zweiten Stift 9.1 passiert hat und eine Schlaufe um den ersten Stift 9.2 gelegt hat, in deren Inneren sich das Bandführungsblech 6 befindet. Die entsprechenden Bereiche Verknotungsbereich und Überschlagsbereich können als Winkel der Umlaufbahn 4 definiert werden.

Die Geschwindigkeit des Mitnehmers 5 ist variabel und der Mitnehmer 5 kann so gesteuert und angetrieben sein, dass er beschleunigt und gebremst werden kann. Befindet sich der Mitnehmer 5 im Verknotungsbereich, so rotiert im Knoter-Aggregat 3 ein Verknotungsdornen (in der Fig. 1 nicht gezeigt) mit einer insbesondere anderthalbfachen Rotation im Gegensinn zur Rotationsrichtung R1 des Mitnehmers 5. Schliesslich durchfährt der Mitnehmer 5 den Wirkbereich einer Schneidklinge 14, welche das Fadenende abtrennt, und so ein Abführen der verknoteten Fadenschlaufe ermöglicht. Der Mitnehmer 5 nimmt ein weiteres Fadenende auf und vollführt die Bewegung in Rotationsrichtung R weiter.

In der **Fig. 2** wird die Bereichseinteilung gemäss Fig. 1 der Wirkbereiche anhand Winkelöffnungen der Umlaufbahn 4 gemäss einer Anordnung wie in Fig. 1 gezeigt nochmals näher erläutert. Die Fig. 2 zeigt schematisch erneut die Umlaufbahn 4 aus der Fig. 1. Ebenfalls gezeigt ist ein Mittelpunkt M der kreisförmigen Umlaufbahn 4. Der Mitnehmer wird auf einem Radius der Umlaufbahn 4 geführt. Ein Prozesszyklus beginnt, wenn der Mitnehmer 5 ein loses Fadenende über einen Umschlagsbereich U führt. Dieser Umschlagsbereich U kann einen ersten Beschleunigungsbereich B1 umfassen. Im Beschleunigungsbereich B1 beschleunigt der Mitnehmer auf eine zweite Geschwindigkeit. Diese Zweite Geschwindigkeit kann mehr aus 500 U/min (relativ zur Umlaufbahn) betragen und bewegt sich im vorliegenden Beispiel im Bereich der 510 U/min. Im Beschleunigungsbereich B1 kann eine Beschleunigung von zwischen 200 und 950 Rad/s² erfolgen.

Die Ausgangsgeschwindigkeit des Mitnehmers 5 zu Beginn des Beschleunigungsbereichs B1 entspricht einer konstanten Geschwindigkeit, die der Mitnehmer während des gesamten Durchfahrens des Verknotungsbereichs V einhält. Die konstante und vergleichsweise tiefere Geschwindigkeit im Verknotungsbereich V ermöglicht es dem Knoter-Aggregat 3 die vom Mitnehmer zugeführten Schlaufenenden zu verknoten. Im vorliegenden Beispiel ist die Geschwindigkeit des Mitnehmers fünf bis sechs Mal geringer während des Verknotungsbereichs V, als die Maximalgeschwindigkeit im Umschlagsbereich U beträgt. Konkret verlässt der Mitnehmer 5 hier den Verknotungsbereich V mit einer Geschwindigkeit von zwischen 50 und 15, insbesondere von etwa 90 U/min.

Um bei erneutem Eintritt in den Verknotungsbereich V sicherzustellen, dass die Geschwindigkeit des Mitnehmers auf die konstante, zwischen halb und sechs Mal geringere Geschwindigkeit relativ zur Maximalgeschwindigkeit des Mitnehmers im Überschlagsbereich U gefahrene Geschwindigkeit gebremst wird, umfasst der Umschlagsbereich U einen zweiten Beschleunigungsbereich B2, de facto einen Bremsbereich B2 in dem die Geschwindigkeit um des Mitnehmers gebremst wird, zum Beispiel mit einer Beschleunigung zwischen 200 und 950 Rad/s².

Im vorliegenden Beispiel kann der Verknotungsbereich einen Winkel von im Wesentlichen 15° umfassen.

Somit besteht je nach Einstellung der Parameter durch den Fachmann ein Zeitintervall im Verknotungsbereich von zwischen 0,015 und 0,05 Sekunden zur Bildung des Knotens. Insgesamt ist die Geschwindigkeit des Mitnehmers in diesem konkreten Ausführungsbeispiel so gewählt, dass er innerhalb eines Zeitraums von zwischen 0,08 und 0,18 Sekunden einen vollständigen Zyklus durchführt. Im vorliegenden Beispiel konnte für den Verknotungsbereich V ein Zeitraum von 0.028 Sekunden definiert werden, und für den Umschlagsbereich U ein Zeitraum vom 0.053 Sekunden bei einer Beschleunigung in den Beschleunigungsbereichen B1, B2 von +, respektive - ca. 940 Rad/s².

Dadurch konnte eine Verknotungsvorrichtung wie eingangs geschildert bereitgestellt werden, die sehr hohe Durchsatzgeschwindigkeiten leistet, und die gleichzeitig mit geringem Materialverschleiss und wenig Wartungsaufwand zu laufen vermag.

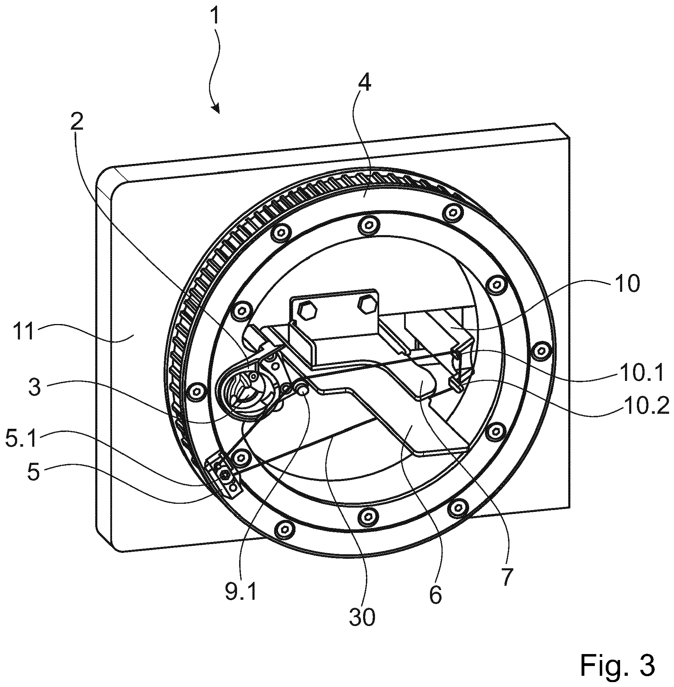

Die **Fig. 3** zeigt eine mögliche Ausgestaltung einer erfindungsgemässen Vorrichtung 1. In diesem Beispiel ist ein Mitnehmer 5 starr mit einer Umlaufbahn 4 verbunden, welche mittels einer Verzahnung über einen Zahnriemen (nicht gezeigt) antreibbar ist. Die Umlaufbahn 4 ist um eine Ringlagerung angeordnet. Der Mitnehmer 5 weist eine Fadenklemme 5.1 auf, welche ein Fadenende 30 greift. Durch seine Bewegung auf der Umlaufbahn 4 haspelt der Mitnehmer 5 Faden von einer (nicht gezeigten) Fadenspule ab. Das Abhaspeln geschieht im vorliegenden Beispiel über die Bewegung des Mitnehmers 5 auf der Umlaufbahn 4, und ist somit kontinuierlich. Ebenfalls denkbar sind aber zwischengeschaltete Ablenkrollen oder eine Abspulrolle, welche den Faden aktiv zum Mitnehmer speist.

Der erste Stift 9.2 ist im vorliegenden Beispiel durch eine Haltevorrichtung 10 ersetzt, welche Haltezähne 10.1, 10.2 aufweist. Diese Haltezähne 10.1, 10.2 halten ein Schlaufenende des Fadenendes 30 offen, so dass ein Auge gebildet wird. Im konkreten Betrieb der Tamponherstellung wird damit die Stirnseite, d.h. das distale eines Rückholfadens gebildet, welcher im Wesentlichen im Inneren des Tampons zu liegen kommt.

Bei der Bewegung des Mitnehmers 5 auf der Umlaufbahn 4 werden die Schenkel so auf die Haltezähne 10.1, 10.2 gelegt, dass die Schlaufe so lange aufgehalten wird, bis ein Verknoten der proximale Schlaufenenden, d.h. die der Stirnseite abgewandten Fadenenden stattgefunden hat. Im Inneren der aufgehaltenen Schlaufe befindet sich ein Bahnführungsblech 6, auf welchem ein bandförmiges Substrat durch den Wirkbereich der Vorrichtung 1 geführt ist. Eine Ablenkfinne 7 ist vorgesehen, um ein Lösen des Fadens zunächst aufzufangen und über ein sich stirnseitig verjüngendes Ende auf das bandförmige Substrat niederzulassen. Die Ablenkfinne 7 ist zunächst so ausgestaltet, dass sie sich parallel zur Förderrichtung des bandförmigen Materials in den Wirkbereich der Vorrichtung erstreckt und stirnseitig verjüngt, um ein sanftes Abgleiten der Schlaufe zu ermöglichen. In besonderen Ausführungsformen kann die Ablenkfinne 7 auswechselbar und/oder verstellbar in der Vorrichtung 1 verbaut werden, um dem Fachmann ein Feinjustieren der Bertriebsparameter an die Fadenlänge, Elastizität etc. zu erleichtern.

Der Mitnehmer 5 führt das Schlaufenende in seiner Bewegung um die Haltezähne, um das Bandführungsblech 6 und um die Ablenkfinne 7 herum an ein Knoter-Aggregat 3 heran. Dort werden beide Schlaufenenden von einer Knoteröhse an einen Verknotungsdorn geführt und verknotet.

Nach dem Knoter-Aggregat kann eine Klinge zur Durchtrennung des Fadenendes vorgesehen sein (nicht gezeigt).

Die gesamte Vorrichtung 1 ist an einem Maschinengestell 11 befestigt.

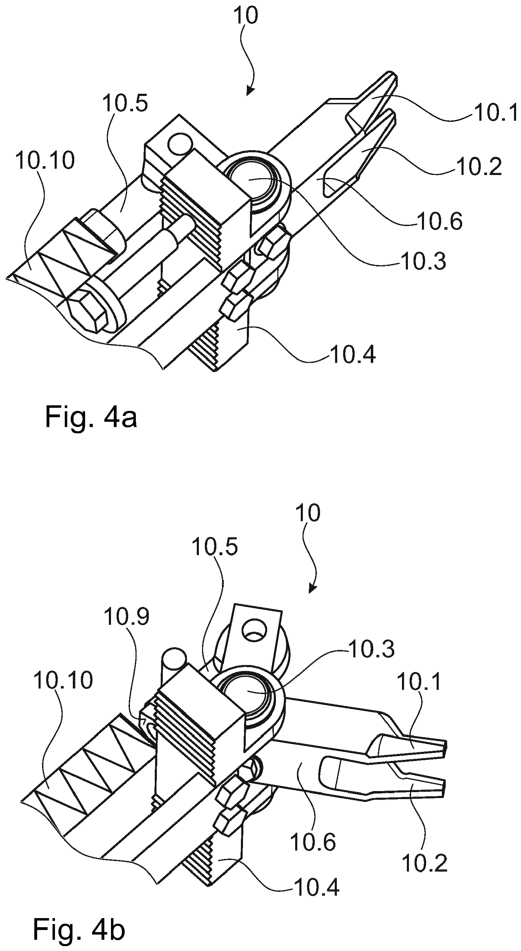

Eine für die erfindungsgemässe Vorrichtung gemäss Fig. 3 verwendbare und als Klinke ausgebildete Haltevorrichtung 10 ist in den **Fig. 4a und 4b** gezeigt. Die Haltevorrichtung 10 verfügt über zwei Haltezähne 10.1, 10.2. Diese dienen dazu, eine Schlaufe, welche vom Mitnehmer 5 bei seiner radialen Bewegung auf der Umlaufbahn um die Haltezähne 10.1, 10.2 gebildet wird, offenzuhalten. Als Klinke ist sind die Haltezähne 10.1, 10.2 auf einem Klinkenarm 1.6 ausgebildet, und um einen Klinkenzapfen 10.3 drehbar gelagert.

Die Haltevorrichtung 10 ist zudem mit einer Sperrschraube und einem Sperrblech 10.4 ausgestaltet und kann in der Vorrichtung so verschiebbar montiert werden. Erneut erleichtert dies dem Fachmann das Justieren der Vorrichtung an die Eigenschaften des Fadenmaterials und eine gewünschte Bandbreite, Geschwindigkeit, Fadenlänge etc. Dazu kann das Sperrblech 10.4 auch über eine Schraube entlang einer Nut verschiebbar in einer Achse zur Bahnspeisung verschiebbar angeordnet sein. Diese Verschiebbarkeit garantiert insbesondere, dass verschiedene Fadenlängen und gewünschte Schlaufengrössen akkommodiert werden können.

Um sicherzustellen, dass der Klinkenarm 10.6 nach einmaligem Auslösen wieder in die ursprüngliche Ausgangslage zurückkehrt, kann eine Rückstellkraft oder ein Rückstellmechanismus vorgesehen sein, wie zum Beispiel eine Feder oder ein Pneumatischer Zylinder.

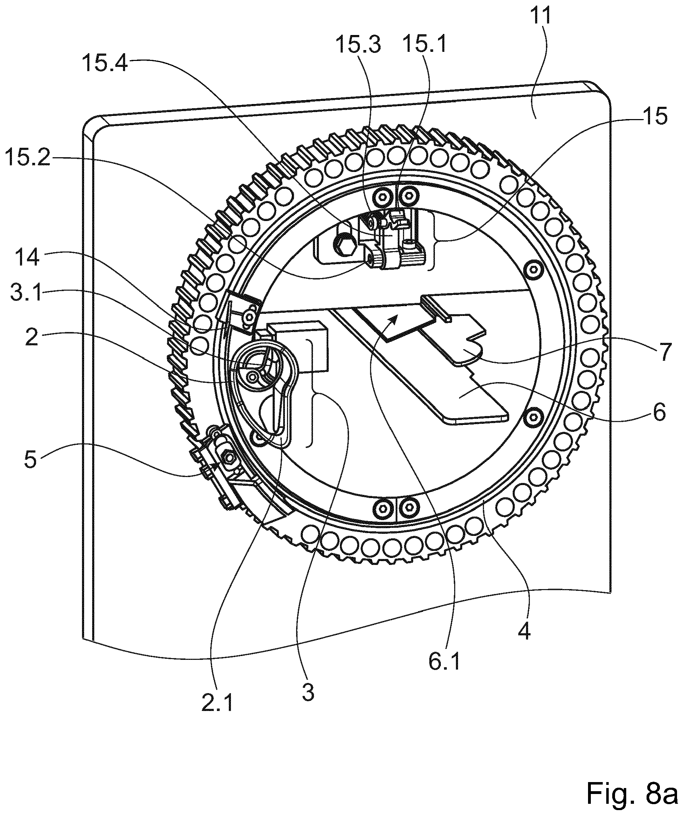

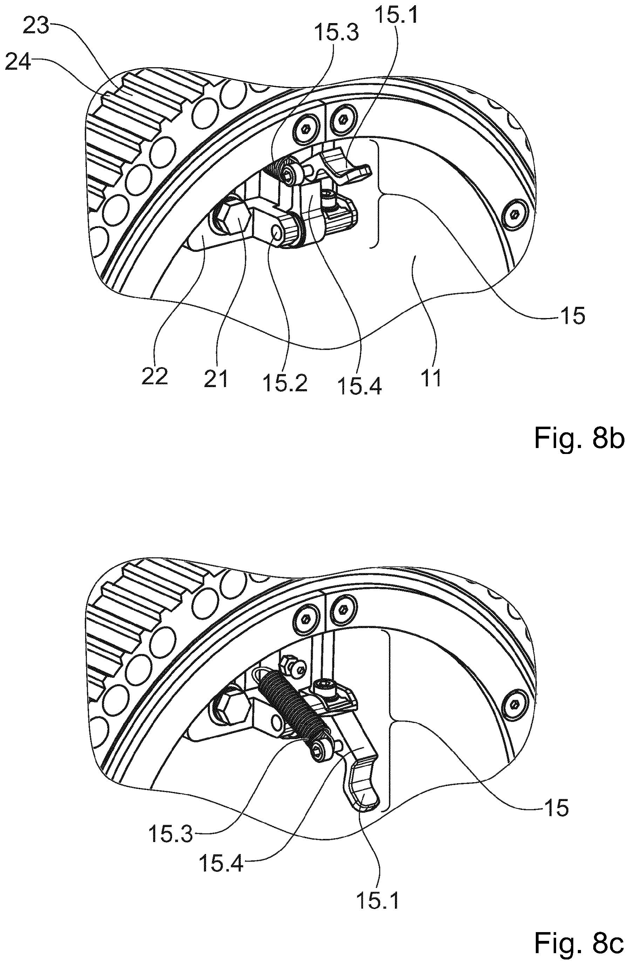

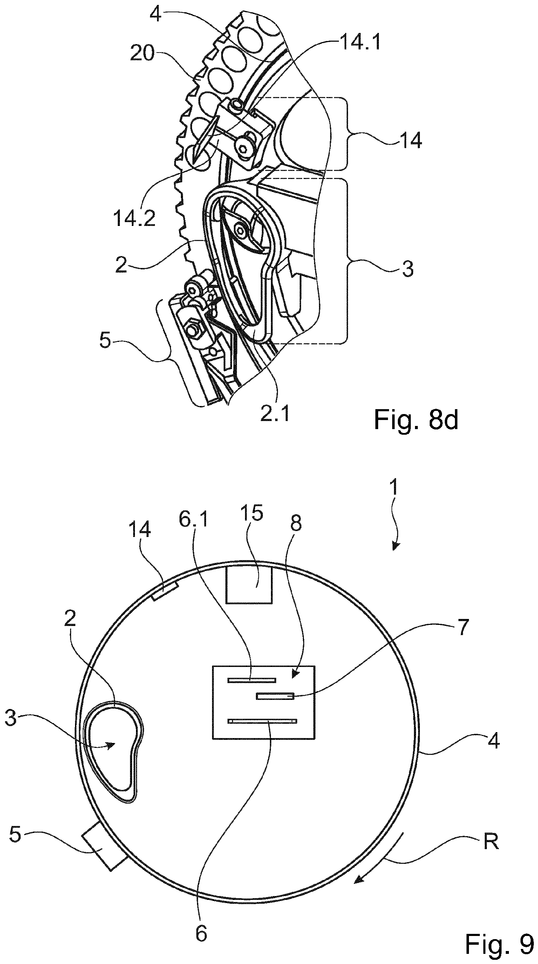

Im vorliegenden Beispiel ist der Klinkenarm 10.10 zudem mit einer Rückstellkraft über einen Dämpfer 10.5 mit einem Anschlag 10.9 gefedert. Durch die Federung ist es möglich, dass der Faden während des Umschlagens nicht reisst. Die Federung wirkt direkt auf das Fadenende des Mitnehmers und gewährleistet ein gewisses Spiel des Fadenendes (siehe auch Federung am Mitnehmer in der Fig.8). Der Faden wird so straff und locker zugleich geführt, ermöglicht genug Spiel für das Führen und Verknoten ohne zu reissen.

In dieser konkreten Ausführungsform ist die Haltevorrichtung so ausgestaltet, dass eine Kraft auf die Fadenschlaufe von zwischen 5 und 25 N, bevorzugt von zwischen 7,5 und 15 N, bevorzugt von im Wesentlichen 10 N ausgeübt werden kann.

An der Fig. 4b ist die Haltevorrichtung der Fig. 4a gezeigt, bei der die Klinke ausgelöst wurde, d.h. der Klinkenarm gelöst ist. In diesem Zustand wird die Schlaufe, die zuvor von den Haltezähnen 10.1, 1.2 gehalten wurde entlassen und der weiteren Prozessführung übergeben. Die Feder 10.10 ist entspannt und der Anschlag 10.9 ist gelöst. Das Lösen hat über eine Rotation des Klinkenhebels 10.6 um den Klinkenzapfen 10.3 stattgefunden. Im Betrieb würde die so ausgestaltete Haltevorrichtung 10 unmittelbar wieder in den Zustand gemäss Fig. 4a überführt, um bereit zu sein um eine neue Fadenschlaufe aufzunehmen. Das kann im vorliegenden Beispiel mit einem Pneumatik-Zylinder der Klinkenhebel wieder in die ursprüngliche Lage zurückgebracht werden. Je nach Einstellungen der Federstärke und der gewünschten Kraft die ausgeübt werden soll auf die Fadenschlaufe von zwischen 5 und 25 N, bevorzugt von zwischen 7,5 und 15 N, bevorzugt von im Wesentlichen 10 N, muss dabei auch der Anschlag 10.9 gegen die Federkraft der entspannten Feder 10.10 gepresst werden.

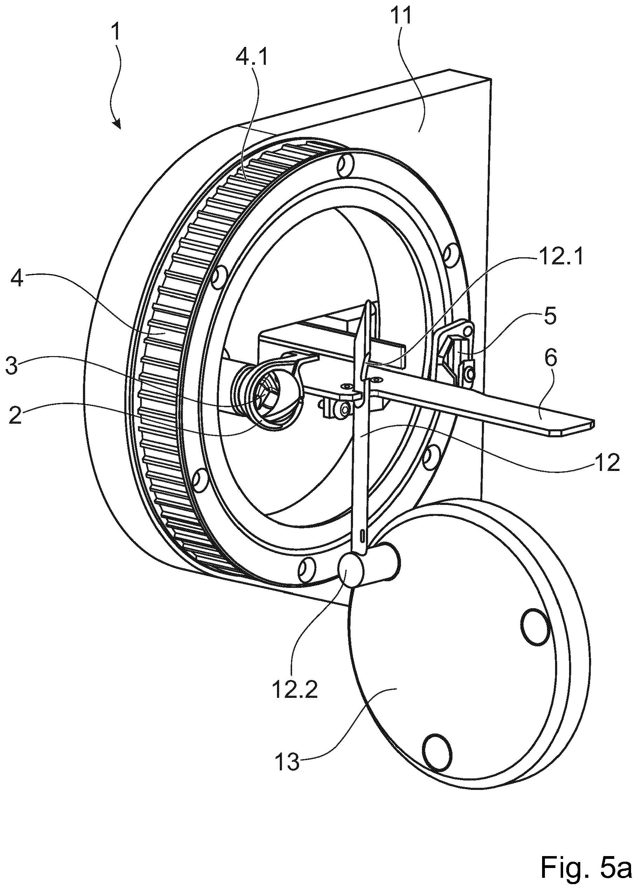

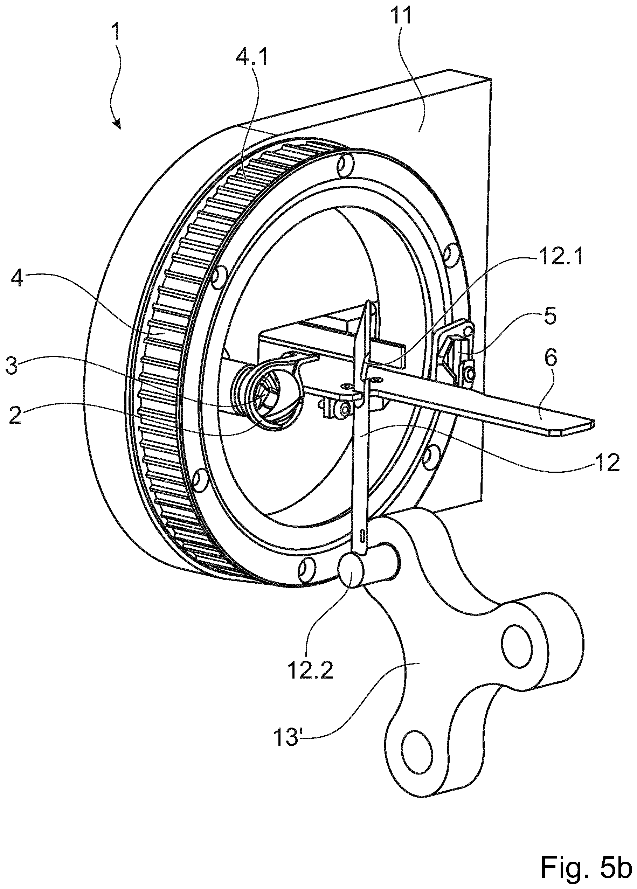

Die **Fig. 5** zeigt eine weitere vorteilhafte Ausgestaltung einer erfindungsgemässen Vorrichtung 1.

Die Vorrichtung 1 umfasst eine Umlaufbahn 4, welche mittels einer Verzahnung 4.1 über einen Zahnriemen antreibbar ist. Dabei ist eine Antriebssteuerung vorgesehen, die so ausgestaltet ist, dass eine Geschwindigkeit der Umlaufbahn 4, welche mittels eines Ringlagers am Maschinengestell 11 gelagert ist, stufenlos verstellbar sein kann.

Insbesondere ist die besagte Antriebssteuerung so ausgestaltet, dass die Umlaufbahn in einem ersten Bereich eine erste Geschwindigkeit und in einem zweiten Bereich eine zweite Geschwindigkeit abzufahren in der Lage ist. Dabei wird ein starr in Rotationsrichtung R (Uhrzeigersinn) der Umlaufbahn verbundener Mitnehmer 5 mitgenommen.

Durch die Vorrichtung erstreckt sich ein Bandführungsblech 6, durch welches ein bandförmiges Material in den Wirkbereich des Mitnehmers 5 gerät und durch den Weg des Mitnehmers 5 um eine Haltevorrichtung 5 und dem Knoter-Aggregat 3 eine Schlaufe sich um das bandförmige Substrat gebildet wird. Um die Schlaufenenden an in einer richtigen Ausrichtung an ein Knoter-Aggregat 3 zu führen, ist eine Knoter-Öhse 2 vorgesehen, welche radial abgeschrägt ist, und in Rotationsrichtung fluchtend ausgestaltet ist.

Die Vorrichtung umfasst weiter einen mittels eines Antriebsrads 13 angetriebener Knoter-Haken 12. Der stiftförmig ausgebildete Knoter-Haken 12 umfasst ein Hakenende 12.1 und ein mit dem Antriebsrad 13 verbundenes und gelagertes Antriebsende 12.2 auf. Im Betrieb wird durch das Hakenende 12.2 in die Schlaufe eingegriffen und neben der Rotationsebene der Umlaufbahn, diese normal zu dieser geöffnet. Die Antriebsscheibe 13 ist zu diesem Zweck rechtwinklig zur Umlaufbahn 4 angeordnet und entsprechend normal zu dieser angetrieben.

Der Knoter-Haken 12 kann in einer besonderen Ausführungsform die Schlaufenenden dem Knoter-Aggregat korrekt zuzuführen und/oder das Abführen der geknüpften Schlaufe zu erleichtern, indem es diese nachdem das Fadenende durchschnitten wurde aus dem Wirkbereich in die Rotationsrichtung des Antriebsrads 13 mittels der Hakenendes 12. Hinausführt.

Das Antriebsrad 13 des Knoter-Haklens 12 ist so angetrieben, dass seine Bewegung und Geschwindigkeit synchron zum Mitnehmer 5 ist. Mit anderen Worten kann das Antriebsrad beispielsweise so ausgestaltet sein, dass es eine ganze Umdrehung vollführt in der Zeit, die benötigt wird, um den Mitnehmer einmal auf der gesamten Umlaufbahn umzuführen.

In einer weiteren zusätzlichen oder alternativen Ausführungsform kann der Knoter-Haken 12 verhindern, dass beim Verknoten durch den Zug des Mitnehmers das Fadenende zu früh in den Wirkbereich des Schneidelements gelangt. In diesem Beispiel kann dies vom Knoter-Haken 12 durch Zug auf die Schlaufe ermöglicht werden, und so das noch nicht fertig verknüpfte Schlaufenende vom Schneidelement fernhalten. In dieser Ausführungsform wäre der Knoter-Haken 12 über das Antriebsrad 13 vorzugsweise synchron zum Knoter-Aggregat 3 getaktet, so dass er eine Umdrehung vollführt in der Zeit, die das Knoter-Aggregat 3 benötigt um einen Knoten zu bilden, also zwischen 1 und 2 Umdrehung zu vollführen.

In der Fig. 5b ist eine weitere vorteilhafte Ausgestaltung der Vorrichtung gemäss Fig. 5 gezeigt. Um insgesamt die bewegte Masse zu verringern, wurde überraschend festgestellt, dass mit einem Antriebsrad 13' mit bevorzugt drei symmetrisch angeordneten Auslegern eine Masseersparnis bei gleichzeitiger hoher Steuerbarkeit und Betriebsruhe erreichbar ist. Auch vier- oder mehrarmige Antriebsräder wären denkbar.

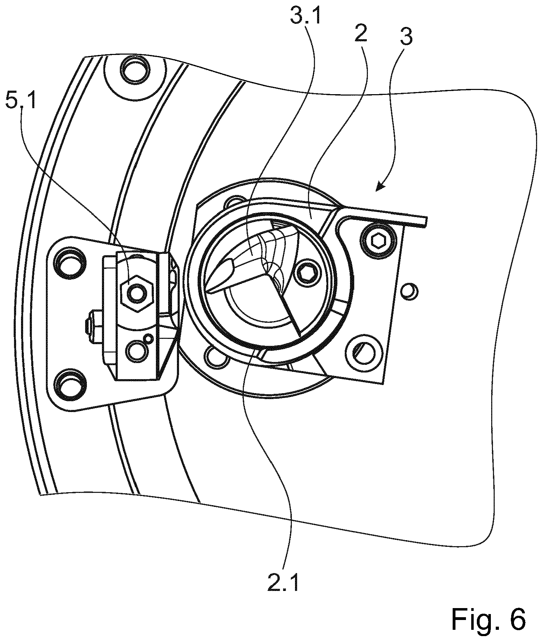

In der **Fig. 6** ist ein Ausschnitt gezeigt einer Vorrichtung wie sie in den Figurenbeschreibungen oben beschrieben wurde, welcher ein geeignetes Knoter-Aggregat 3 und einen Mitnehmer 5 zeigt.

Der gezeigte Mitnehmer 5 ist starr mit einer Umlaufbahn 4 verbunden und zu Illustrationszwecken direkt neben einem Knoter-Aggregat 3 befindlich. Diese Stellung kann der Mitnehmer zum Beispiel bei Beginn des Eintritts in den Verknotungsbereich aufweisen.

Die Schlaufenenden des Fadenendes sind der vereinfachten Darstellung halber nicht gezeigt.

Das Knoter-Aggregat 3 ist innerhalb einer Knoter-Öhse 2 angeordnet und umfasst einen Verknotungsdornen 3.1, welcher ausgelegt ist, innerhalb der Knoter-Öhse 2 im Gegenuhrzeigersinn zu rotieren. Dabei vollführt der Verknotungsdorn 3.1 eine mit einem Spitzen Ende zu den Fadenschlaufen verlaufende Tauchbewegung und greift in von Mitnehmer und Haltevorrichtung gehaltenen Schlaufenenden ein. Durch den Andruck der Schlaufenenden, die Rotation des Verknotungsdorns und eine Führung über die Knoter-Öhse wird in einer 1- bis 2- fachen, bevorzugt anderthalbfachen Umdrehung des Verknotungsdorns ein Knoten gebildet.

Die Knoter-Öhse 2 umfasst eine Führungsgeometrie 2.1, welche nach aussen hin, d.h. in radialer Richtung, fluchtend ist. Im Betrieb werden ein Schlaufenende zunächst an die Führungsgeometrie 2.1 herangeführt und im weiteren Verlauf der Bahn des Mitnehmers 5 in den Wirkbereich des Verknotungsdornens 3.1 geführt, welcher in umgekehrter Richtung zur Umlaufbahn des Mitnehmers 5 rotiert.

Im vorliegenden Beispiel hat der Mitnehmer 5 einen mit einer Rückstellkraft versehenen Mitnahmegreifer 5.1, durch welchen die Schlaufe mit ausreichend Spiel in die Verknotung geführt wird.

Schliesslich gelangt das zweite Schlaufenende in den Wirkbereich des Verknotungsdornens 3.1 und wird mit dem ersten Schlaufenende verknotet.

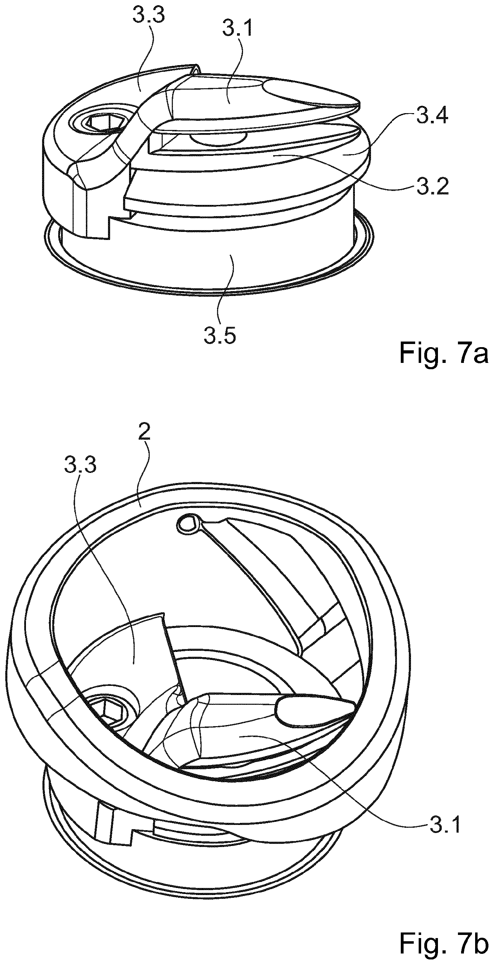

In der **Figur 7a** wird zur Verdeutlichung das Knoter-Aggregat der Figur 6 ohne Knoter-Öhse in Seitenaufnahme schematisch gezeigt. Das Knoter-Aggregat umfasst im Wesentlichen einen austauschbaren Verknotungsdorn 3.1, der über einen Dornfuss 3.3 lösbar und starr mit einer rotierbar gelagerten Antriebsscheibe 3.5 verbunden ist. Insgesamt ist das gezeigte Knoter-Aggregat 3 mehrteilig ausgebildet. Der Verknotungsdorn 3.1 umfasst einen ersten Dornfortsatz 3.1 und einen parallel ausgerichteten zweiten Dornfortsatz 3.2, sowie eine ebenfalls parallel zu den Verknotungsdornfortsätzen ausgerichtete Fadenführungsblende 3.4. Im Betrieb wird durch die zweiteilige Dornenform und die Fadenführungsblende 3.4 gewährleistet, dass die verknoteten Fadenschlaufen aus dem Knoter-Aggregat nach dem Verknoten abgeführt werden können.

Wie in der **Figur 7b** ersichtlich, verfügt die seitlich abgeschrägte und radial fluchtend ausgestaltete Knoter-Öhse 2 über eine entsprechende Ausnehmung.

Geeignet für die erfindungsgemässe Vorrichtung ist ein in Rotationsrichtung starr mit der Umlaufbahn verbundener Mitnehmer. Der Mitnehmer kann, zum Beispiel, mittels einer Spannschraube, oder einer Mehrzahl an Spannschrauben, welche sich durch das gesamte Profil der Umlaufbahn erstrecken mit der Umlaufbahn lösbar verbunden sein. Dazu können beispielsweise in der Umlaufbahn vorgebohrte Ausnehmungen vorgesehen sein.

Ein erstes Federelement kann ebenso am Mitnehmer vorgesehen sein, der die gesamte Profildicke der Umlaufbahn durchspannt, und ein rückseitiges Element des Mitnehmers mit einem vorderseitigen Element des Mitnehmers verbindet. Das vorderseitige Element des Mitnehmers wäre im vorliegenden Beispiel dasjenige Element, welches das Fadenende in den Wirkbereich der Haltevorrichtung und des Verknoter-Aggregats führt. Dadurch kann das erste Federelement eine Wirkverbindung zwischen auf der Rückseite angeordneter Federn und einer auf der Vorderseite vorgesehenen Fadenklemme erzeugen. Im Betrieb würde dies dazu führen, dass durch die Federung die Fadenklemme etwas Spiel für das Fadenende zulässt. Gemeinsam zum Beispiel mit der oben geschilderten gefederten Haltevorrichtung kann dadurch das Verknoten durch das Verknoter-Aggregat erleichtert, ein Reissen eines Fadenendes verhindert und höhere Prozessgeschwindigkeiten ermöglicht werden.

Die **Figur 8a** illustriert einen weiteren Aspekt, respektive eine besondere Ausführungsform der vorliegenden Erfindung. Die gezeigte Vorrichtung ist geeignet, um ein Fadenende um ein bandförmiges Substrat, zum Beispiel ein Watteband für einen Tampon, zu legen und zwei Fadenstrecken dieses Fadenendes zu Verknoten, so dass eine Schlaufe entstehen kann. Wird dieses Watteband aufgerollt, gewickelt und/oder gefaltet, so können diese Fadenstrecken des Fadenendes aus einem resultierenden Tampon herausragen und als Rückholfaden dienen. Bei der Verknotung des Fadenendes wird also eine Schlaufe um ein bandförmiges Substrat gebildet. Das Fadenende (nicht gezeigt) wird von einem Mitnehmer 5 abgespult. Der Mitnehmer 5 ist starr mit einem gezahnten Umlaufrad 20 verbunden. Dieses Umlaufrad 20 kann z.B. von einem Antriebsriemen, oder einem Antriebsrad angetrieben sein (nicht gezeigt), und ist bevorzugt beschleunigbar, respektive bremsbar. Das Umlaufrad 20 ist an einer Umlaufbahn 4 gelagert, so dass der Mitnehmer 5 eine kreisförmige Bewegung zu vollführen vermag. Dabei führt der Mitnehmer das Fadenende an verschiedenen Bearbeitungseinheiten vorbei.

Bei einer vollständigen Rotation um die Umlaufbahn 4, wird der Faden einmal um ein mittig in den Wirkbereich des Mitnehmers gefördertes Substratband, z.B. Watteband gelegt. Dabei wird das Fadenende zunächst um einen Abnehmer gelegt, welcher das Fadenende und die resultierende Schlaufe zunächst offenhält. Weitere Fadenstrecke des Fadenendes wird auf eine Ablenkfinne gelegt.

Das offene Fadenende wird anschliessend an ein Knoter-Aggregat 3, wo zwei Fadenstrecken des Fadenendes miteinander verknüpft werden. Eine auf einer Knoter-Öhse ausgebildete Führungsgeometrie 2.1 stellt sicher, dass das freie Fadenstück korrekt zum zu verknotenden Fadenstück geführt wird.

Die so erzeugte Schlaufe wird in der Normale zur Bildebene mitsamt des Wattebands abgeführt. Der Abnehmer 15 lässt die erzeugte Schlaufe kontrolliert auf das Substratband hinabgleiten, indem die in einem Abnehmerhaken 15.1 gehaltene Fadenstrecke durch eine Senkung eines Abnehmerhebels 15.4 gelöst wird. Zu diesem Zweck ist der Abnehmerhebel 15.4 schwenkbar in einem Gelenk 15.2 gelagert. Eine Abnehmerfeder 15.3 übt eine Rückstellkraft auf den Abnehmerhaken 15.1 aus. Diese wird durch den Verknotungsprozess des Knoter-Aggregats 3 und entsprechendem Zug auf das Fadenende überwunden. Befindet sich der Abnehmerhebel 15.4 in seiner maximal gesenkten Position, entlässt er die Fadenstrecke, womit die Rückstellkraft diesen wieder in die angewinkelte Ausgangsposition zurückführt.

Nach dem Verknoten wird das Fadenende an der Schneidklinge 14 vorbeigeführt, womit die Schlaufe abgetrennt wird. Der Mitnehmer 5 beginnt eine neue Umdrehung auf der Umlaufbahn 4 mit einem neuen Fadenende. Die genannten Elemente sind ist an einem Maschinenrahmen 11 angebracht, durch welchen durch zentral vorgesehene Ausnehmungen einen Bandeinlass für das Substratband ermöglichen.

Das Substratband ist horizontal durch ein Bandführungsblech 6 geführt. Zusätzlich zum Bandführungsblech ist ein parallel zu diesem ist ein Anschlagblech 6.1 ausgebildet, welches verhindert, dass der Abnehmerhaken mit der Bandförderung des bandförmigen Substrates interferiert.

Eine Ablenkfinne 7 führt das Fadenende von der gegenüberliegenden Seite nahe am Watteband und ermöglicht ein sanftes Aufgleiten der erzeugten Schlaufe durch ihr sich in Förderrichtung des Wattebandes, also zur Betrachterebene hin verjüngendes Ende. Zusätzlich übt die Ablenkfinne durch eine Einkerbung den erforderlichen Widerstand für das Knoter-Aggregat zum Verknoten der Fadenenden. Nachdem die Schlaufe abschliessend verknotet ist und ein Knoterhaken (siehe Fig. 5a oder 5b) die erzeugte Schlaufe mit dem Substratband abführt, führt die Ablenkfinne 7 durch die sich verjüngende Form die Schlaufe auf das bandförmige Substrat. Durch eine bauchige Ausformung im Anschluss an die Einkerbung wird auch der erforderliche Zugwiderstand sichergestellt, um die Schlaufe aus der Knoter-Öhse 2 zu lösen

Mit der gezeigten Vorrichtung und der resultierenden sanften Ablage der Fadenschlaufe auf das Watteband kann eine hohe Prozessgeschwindigkeit mit hoher Zuverlässigkeit erreicht werden. Diese Vorrichtung ist ebenso geeignet eine entsprechende Beschleunigung und variable Geschwindigkeit des Mitnehmers 5 zu fahren, wie in der Ausführungsform der Fig. 1 bis 7 oben beschrieben.

Die **Fig. 8b** zeigt ausschnittsweise den Abnehmer 15 am oberen Ende der Umlaufbahn. Der Abnehmer 15 ist so positioniert, dass er einem Winkel von nicht mehr als 95 und nicht weniger als 85 °Grad bezüglich der Ebene des Bandführungsblechs (in dieser Figur nicht gezeigt) und somit zum bandförmigen Substrat einnimmt. Dabei ist der Winkel von der Mitte der Stirnseite des Abnehmerhakens 15.1 aus betrachtet. Der Abnehmerhaken 15.1 ist am Ende eines Abnehmerhebels 15.4 im Wesentlichen hakenförmig ausgebildet und eignet sich zur Aufnahme einer Fadenstrecke des Fadenendes (nicht gezeigt). Der Abnehmerhebel 15.4 ist um ein Gelenk 15.2 schwenkbar gelagert, und in der Figur 8b angewinkelt gezeigt. Eine Abnehmerfeder ist zwischen Abnehmerhebel 15.4 und Maschinenrahmen 11 wirkverbunden, so dass eine Rückstellkraft auf den Abnehmerhebel 15.4 wirkt. Der Abnehmer 15 ist mit dem Maschinenrahmen 11 über einen Flansch 22 mit einer Schraube 21 verschraubt. Um den Abnehmer 15 führt radial eine Umlaufbahn. In der Fig. 8b sind Nuten 23 und Zähne 24 zu sehen, welche zum Beispiel mit einem Antriebsband oder Riemen, z.B. einem Raupenband in Wirkverbindung gebracht werden können.

In der **Fig. 8c** ist der in der Figur 8b gezeigte Abnehmerhebel 15.4 in abgewinkelter Haltung gezeigt, bei dem der Abnehmerhaken 15.1 die zuvor gehaltene Fadenstrecke entlässt und auf ein Substratband gleiten lässt. Die Abnehmerfeder 15.3 ist voll gestreckt und übt die volle Rückstellkraft auf den Abnehmerhebel aus. Hat die Fadenstrecke den Abnehmerhaken verlassen, schnellt der Abnehmerhebel zurück in die angewinkelte Haltung der Fig. 8b, bereit ein neues Fadenstück aufzunehmen.

Die **Figur 8d** zeigt einen anderen Bereich der Ausführungsform der Figur 8a im Detail. Gezeigt sind ein Knoter-Aggregat 3, ein Mitnehmer 5, sowie eine Schneidklinge 14. Der Mitnehmer 5 ist im Betrieb ausgelegt, um ein Fadenende abzuspulen, und es auf der Umlaufbahn der Vorrichtung zu einer Schlaufe zu formen. Dabei werden zwei Fadenstrecken im Knoter-Aggregat 3 so zueinander geführt, dass diese zu einem Knoten verknotetet werden können. Das Knoter-Aggregat 3 ist ein komplexes Bauteil, welches Aussen von einer Knoter-Öhse 2 umgeben ist. Die Knoter-Öhse 2 weist der der Rotationsrichtung zugewandten Seite eine Führungsgeometrie 2.1 auf, welche der Aufnahme des freien Fadenstücks dient. Oberhalb des Knoter-Aggregats 3 ist eine Schneidklinge 14 vorgesehen, die fest mit dem Maschinenrahmen verbunden ist über eine Schneidklingenhalterung 14.2, die wiederum eine auswechselbar ausgestaltete Klinge 14.1 in den Wirkbereich des Mitnehmers hält, so dass eine Schlaufe aus dem Fadenende abgeschnitten wird, wenn der Mitnehmer die Schneidklinge passiert. Der Mitnehmer ist starr mit einem Umlaufrad verbunden, welches wie bereits geschildert z.B. über einen Riemen antreibbar ist.

Weiter schematisch ist die Funktionsweise der Ausführungsform 8a-d in der **Figur 9** nochmals dargestellt.

Die Vorrichtung 1 umfasst eine Umlaufbahn 4, um welche ein Mitnehmer 5 bewegbar gelagert ist. Der Mitnehmer 5 vollführt also eine kreisförmige Bewegung um einen im Wesentlichen mittig in der Vorrichtung 1 vorgesehenen Bahneinlass 8, der dazu dient ein bandförmiges Substrat im Wesentlichen senkrecht zur Bildebene in den Wirkbereich des Mitnehmers 5 zu fördern.

Bei der Bewegung auf seiner Umlaufbahn 4 führt der Mitnehmer ein Fadenende mit, dessen Fadenstrecken mit den einzelnen Elementen der Vorrichtung 1, die entlang der Umlaufbahn angeordnet sind in Wechselwirkung gelangen. Zunächst wird eine Schleife um den Bahneinlass 8 mit dem Fadenende gebildet. Zwei Fadenstrecken des Fadenendes werden im Knoter-Aggregat 3 durch eine Knoter-Öhse 2 so aneinander geführt, dass ein Verknotungsdorn (nicht gezeigt) diese zu Verknoten vermag. Dabei wird eine Fadenstrecke von einem Abnehmer 15, der im Wesentlichen in einem rechten Winkel oberhalb des Substratbandes angeordnet ist, gehalten. Anschliessend wird die Fadenstrecke durch den Abnehmer 15 gegen eine Rückstellkraft sanft auf das Substratband abgesenkt. Ein Anschlagsblech 6.1 kann oberhalb des bandförmigen Substrates vorgesehen sein, damit der Abnehmer 15 dieses nicht berührt, und so den Lauf des bandförmigen Substrates stören könnte. Das bandförmige Substrat wird am Bahneinlass 8 von einem Führungsblech 6 geführt.

Eine auf der Umlaufbahn 4 fest angeordnete Schneidklinge 14 trennt die erzeugte Schlaufe ab, welche mitsamt des Substratbandes abgeführt wird, insbesondere z.B. durch einen Knoter-Haken 12, wie er in den Fig. 5a oder 5b gezeigt ist. Die gebildete Schlaufe wird aus der Knoter-Öhse des Knoter-Aggregats gelöst, und gleitet über eine sich verjüngende Ablenkfinne 7 stirnseitig auf das bandförmige Substrat. Mit dem Abfördern des bandförmigen Substrates wird die Schlaufe mitgezogen und kann bei einem mittels Wickeln, Falten und/oder Pressen erzeugten Tampon als Rückholfaden herausragen.

Mit der vorliegenden Erfindung ist eine Vorrichtung zum Verknoten loser Fadenenden zur Herstellung von Tampons mit Rückziehfäden bereitgestellt, welche sicher ist im Betrieb sowie leicht zu warten und in der Lage, eine kontinuierliche Tamponproduktion mit hohen Stückzahlen bereitzustellen.

### Bezugszeichenliste

- 1: Vorrichtung zur Verknotung eines Fadenendes
- 2: Knoter-Öhse
- 2.1: Führungsgeometrie
- 3: Knoter-Aggregat
- 3.1: Verknotungsdorn
- 3.2: zweiter Dornfortsatz
- 3.4: Fadenführungsblende
- 3.5: rotierbar gelagerte Antriebsscheibe
- 4: Umlaufbahn
- 5: Mitnehmer
- 5.1: Mitnahmegreifer
- 6: Führungsblech
- 6.1: Anschlagsblech

- 7: Ablenkfinne
- 8: Bahneeinlass
- 9.2: erster Stift
- 9.1: zweiter Stift
- 10: Haltevorrichung
- 10.1: erster Haltezahn
- 10.2: zweiter Haltezahn
- 10.3: Klinkenzapfen
- 10.4: Sperrblech
- 10.5: Dämpfer
- 10.6: Klinkenarm
- 10.9: Anschlag
- 10.10: Feder

- 11: Maschinengestell
- 12: Knoter-Haken
- 12.1: Hakenende
- 12.2: Antriebsende
- 13: Antriebsrad
- 14: Schneidklinge
- 14.1: Klinge
- 14.2: Schneidklingenhalterung
- 15: Abnehmer
- 15.1: Abnehmerhaken
- 15.2: Gelenk
- 15.3: Abnehmerfeder
- 15.4: Abnehmerhebel

- 20: Umlaufrad
- 23: Nut
- 24: Zahn

- 30: Fadenende
- B1: Beschleunigungsbereich
- B2: zweiter Beschleundigungsbereich
- M: Mittelpunkt
- V: Verknotungsbereich
- R: Rotationsrichtung
- U: Umschlagsbereich

## Patentansprüche

1. Vorrichtung (1) zur Verknotung eines Fadenendes so dass eine Schlaufe gebildet wird, insbesondere eines Fadenendes eines Rückholfadens für Tampons, umfassend:
a. Ein **Knoter-Aggregat** (3) zur Verknotung von zwei Fadenstrecken des Fadenendes;
b. Einen **Mitnehmer** (5) zum Zuführen der Fadenstrecken an das Knoter-Aggregat (3);
c. Eine **Umlaufbahn** (4), entlang welcher der Mitnehmer um das Knoter-Aggregat (3) geführt wird, und
**dadurch gekennzeichnet, dass**
die Umlaufbahn einen **Verknotungsbereich** (V) aufweist, in dem der Mitnehmer mit einer **ersten Geschwindigkeit** bewegbar ist, und einen **Umschlagsbereich** (U) aufweist, in dem der Mitnehmer mit einer **zweiten Geschwindigkeit** bewegbar ist, und
weiter umfassend eine als Klinke ausgebildete Haltevorrichtung (10), die ausgelegt ist eine Rückstellkraft auf ein Schlaufenende des zu verknotenden Fadenendes auszuüben.

2. Vorrichtung gemäss Anspruch 1, weiter umfassend eine **Fadenspeisung** zur kontinuierlichen Zufuhr eines Fadenendes an den Mitnehmer.

3. Vorrichtung gemäss einem der Ansprüche 1 oder 2, weiter umfassend ein **Schneidelement** zum Abtrennen der Schlaufe des Fadenendes.

4. Vorrichtung gemäss einem der Ansprüche 1 bis 3, wobei die Umlaufbahn eine im Wesentlichen kreisförmige Umlaufbahn ist.

5. Vorrichtung gemäss einem der Ansprüche 1 bis 4, umfassend einen **Bahneinlass** zur Durchfuhr eines bandförmigen Substrats durch den Wirkbereich des **Mitnehmers**, so dass das Fadenende um das bandförmige Substrat gelegt wird, insbesondere so dass bei einem Umlauf des Mitnehmers auf der Umlaufbahn das Fadenende um das bandförmige Substrat gelegt wird.

6. Vorrichtung gemäss Anspruch 5, weiter umfassend ein Führungsblech zum Führen bandförmigen Substrats durch den Wirkbereich des **Mitnehmers.**

7. Vorrichtung gemäss einem der Ansprüche 1 bis 6, weiter umfassend eine Knotenkontrolle zur Überprüfung des vom Knoter-Aggregat (3) geknüpften Knotens von zwei Fadenstrecken.

8. Vorrichtung gemäss einem der Ansprüche 1 bis 7, wobei die Umlaufbahn antreibbar ist, insbesondere eine Verzahnung aufweist, durch welche die Umlaufbahn mittels eines Zahnriemens antreibbar ist, so dass sie eine beschleunigbare Rotation um eine Rotationsachse auszuführen vermag.

9. Vorrichtung nach einem der Ansprüche 1 bis 7, umfassend einen Direktantrieb zum Antreiben des Mitnehmers, insbesondere einen elektrischen Direktantrieb.

10. Vorrichtung gemäss einem der Ansprüche 1 bis 9, weiter umfassend einen **Umlaufantrieb**, insbesondere einen stufenlos beschleunigbaren Umlaufantrieb, zum Antrieb des Mitnehmers auf der Umlaufbahn.

11. Vorrichtung gemäss einem der Ansprüche 1 bis 10, wobei das Knoter-Aggregat einen **Verknotungsdornen** umfasst, der rotierbar angetrieben ist.

12. Vorrichtung gemäss einem der Ansprüche 1 bis 11, wobei die Vorrichtung ausgestaltet ist, um den Mitnehmer mit einer ersten Geschwindigkeit durch einen Verknotungsbereich zu führen, wobei die erste Geschwindigkeit tiefer ist als eine zweite Geschwindigkeit, mit der der Mitnehmer durch den Umschlagsbereich geführt wird, insbesondere wobei die erste Geschwindigkeit eine Höchstgeschwindigkeit hat, die um zwischen zwei und neunmal tiefer ist als eine Höchstgeschwindigkeit der zweiten Geschwindigkeit.

13. Vorrichtung gemäss einem der Ansprüche 1 bis 12, wobei die erste Geschwindigkeit zwischen 300 und 600 °/s liegt und die zweite Geschwindigkeit zwischen 500 und 4000 °/s liegt.

14. Vorrichtung gemäss Anspruch 13, wobei die Vorrichtung ausgestaltet ist, um den Mitnehmer mit einer ersten Geschwindigkeit durch einen Verknotungsbereich von zwischen 50 und 150 U/min, insbesondere etwa 90 U/min zu führen, und den Mitnehmer mit einer zweiten Geschwindigkeit durch Umschlagsbereich von zwischen 200 U/min und 600 U/min, insbesondere von ca. 500 U/min zu führen.

15. Vorrichtung gemäss einem der Ansprüche 1 bis 14, weiter umfassend eine Antriebssteuerung zur Bewegung des Mitnehmers im Verknotungsbereich mit einer ersten Geschwindigkeit, und in einem Umschlagsbereich (U) mit einer zweiten Geschwindigkeit, insbesondere wobei die Antriebssteuerung ausgelegt ist den Mitnehmer mit einer ersten Geschwindigkeit durch den Verknotungsbereich zu führen, so dass dieser den Verknotungsbereich verlässt nachdem das Knoter-Aggregat zwischen einer und zwei, insbesondere im Wesentlichen anderthalb Umdrehungen entgegen der Richtung des Mitnehmers durchgeführt hat.

16. Verfahren zur Verknotung eines Fadenendes, insbesondere von zwei Fadenstrecken des Fadenendes, so dass eine Schlaufe entsteht, welche ein Rückholfaden für Tampons bildet, umfassend die Schritte:
a. **Führen** eines Fadenendes eines Faden mittels eines auf einer Umlaufbahn bewegbaren Mitnehmers entlang einer Umlaufbahn um ein Knoter-Aggregat und einen **Umschlagselement**, so dass zwischen Knoter-Aggregat und Umschlagselement eine Schlaufe aus zwei Fadenstrecken des Fadenendes gebildet ist;
b. **Verknoten** der zwei Fadenstrecken durch das Knoter-Aggregat und **Lösen** der Schlaufe am Umschlagselement, und
wobei die Umlaufbahn Verknotungsbereich (V) aufweist, in dem der Mitnehmer mit einer ersten Geschwindigkeit geführt wird, und einen Umschlagsbereich (U) aufweist, in dem der Mitnehmer mit einer zweiten Geschwindigkeit geführt wird, und
wobei das Lösen der Schlaufe am Umschlagselement ein Lösen einer Klinke vorsieht, welche ausgestaltet ist eine lösbare Rückstellkraft in Richtung des Mitnehmers und/oder Knoter-Aggregats auszuüben.

17. Verfahren gemäss Anspruch 16, wobei
a. eine Zufuhr eines Fadenendes von einem Faden auf einer Spule auf dem über die Umlaufbahn bewegbaren Mitnehmer über eine Rückstellkraft geschieht, und
b. das Lösen der Schlaufe ein Abschneiden des Fadenendes von dem Faden umfasst.

18. Verfahren gemäss einem der Ansprüche 15 oder 16, wobei das Verhältnis der ersten Geschwindigkeit zur zweiten Geschwindigkeit zwischen 1:2 und 1:6 beträgt.

19. Verfahren gemäss einem der Ansprüche 16 bis 18, wobei der Verknotungsbereich (V) einen Winkel von zwischen 5 und 45 Grad der Umlaufbahn einschliesst, bevorzugt zwischen 5 und 30 Grad einschliesst, bevorzugt 15 Grad einschliesst.

20. Verfahren gemäss einem der Ansprüche 16 bis 19, weiter umfassend die Schritte:
a. **bereitstellen** einer Vorrichtung (1) gemäss Anspruch 1 zur Verknotung eines Fadenendes so dass eine Schlaufe gebildet wird;
b. **durchfuhr** eines bandförmigen Substrats durch einen Wirkbereich des Mitnehmers, so dass das Fadenende um das bandförmige Substrat gelegt wird, insbesondere so dass bei einem Umlauf des Mitnehmers auf der Umlaufbahn das Fadenende um das bandförmige Substrat gelegt wird, wobei die Durchfuhr des bandförmigen Substrats im Wesentlichen normal zu einer Umlaufbahn eines Mitnehmers angeordnet ist.

21. Vorrichtung zur Herstellung von Tampons mit einem proximalen Rückholfaden umfassend zwei miteinander verknüpfte Fadenenden, die Vorrichtung umfassend:
a. Eine Vorrichtung zur Verknotung zweier Fadenenden gemäss einem der Ansprüche 1 bis 15,
b. Eine **Fördervorrichtung** zur Förderung von bandförmigem Substrat in einen Wirkbereich der Vorrichtung zur Verknotung zweier Fadenenden, insbesondere wobei die Fördervorrichtung im Wesentlichen normal zu einer Umlaufbahn eines Mitnehmers zum Zuführen der zwei Fäden an ein Knoter-Aggregat angeordnet, und
wobei die Fördervorrichtung zur Beförderung des bandförmigen Substrats dergestalt in den Wirkbereich der Vorrichtung zur Verknotung zweier Fadenenden fördert, dass bei einer Umlaufbewegung eines Mitnehmers entlang einer Umlaufbahn der Faden im Wesentlichen quer zur Längsachse des bandförmigen Substrats um dieses gelegt wird.

## Claims

1. A device (1) for knotting a thread end such that a loop is formed, in particular a thread end of a retrieval thread for tampons, comprising:
a. a **knotter assembly** (3) for knotting two thread sections of the thread end;
b. a **driver** (5) for feeding the thread sections to the knotter assembly (3);
c. an **orbital path** (4) along which the driver is guided around the knotter assembly (3), and
**characterized in that**
the orbital path has a **knotting region** (V) in which the driver is movable at a **first speed**, and an **envelope region** (U) in which the driver is movable at a **second speed**, and
further comprising a holding device (10), designed as a latch, which is designed to exert a restoring force on a loop end of the thread end to be knotted.

2. The device according to claim 1, further comprising a **thread feed** for continuously feeding a thread end to the driver.

3. The device according to any of claims 1 or 2, further comprising a **cutting element** for severing the loop of the thread end.

4. The device according to any of claims 1 to 3, wherein the orbital path is a substantially circular orbital path.

5. The device according to any of claims 1 to 4, wherein a **path inlet** for passing a strip-shaped substrate through the active region of the **driver** such that the thread end is laid around the strip-shaped substrate, in particular such that the thread end is laid around the strip-shaped substrate when the driver orbits on the orbital path.

6. The device according to claim 5, further comprising a guide plate for guiding the strip-shaped substrate through the active region of the **driver**.

7. The device according to any of claims 1 to 6, further comprising a knot control for checking the knot of two thread sections tied by the knotter assembly (3).

8. The device according to any of claims 1 to 7, wherein the orbital path is drivable, in particular comprises teeth by means of which the orbital path is drivable by means of a toothed belt so as to be capable of performing acceleratable rotation about an axis of rotation.

9. The device according to any of claims 1 to 7, comprising a direct drive for driving the driver, in particular an electric direct drive.

10. The device according to any of claims 1 to 9, further comprising an **orbital drive**, in particular a continuously acceleratable orbital drive, for driving the driver on the orbital path.

11. The device according to any of claims 1 to 10, wherein the knotter assembly comprises a **knotting mandrel** which is rotatably driven.

12. The device according to any of claims 1 to 11, wherein the device is configured to guide the driver through a knotting region at a first speed, wherein the first speed is lower than a second speed at which the driver is guided through the envelope region, in particular wherein the first speed has a maximum speed that is between two and nine times lower than a maximum speed of the second speed.

13. The device according to any of claims 1 to 12, wherein the first speed is between 300 and 600 °/s and the second speed is between 500 and 4000 °/s.

14. The device according to claim 13, wherein the device is configured to guide the driver through a knotting region at a first speed of between 50 and 150 rpm, in particular approximately 90 rpm, and to guide the driver through an envelope region at a second speed of between 200 rpm and 600 rpm, in particular approx. 500 rpm.

15. The device according to any of claims 1 to 14, further comprising a drive control system for moving the driver in the knotting region at a first speed, and in an envelope region (U) at a second speed, in particular wherein the drive control system is designed to guide the driver through the knotting region at a first speed such that this leaves the knotting region after the knotter assembly has performed between one and two revolutions, in particular substantially one and a half revolutions, counter to the direction of the driver.

16. A method for knotting a thread end, in particular two thread sections of the thread end, such that a loop is formed which forms a retrieval thread for tampons, comprising the steps of:
**a. guiding** a thread end of a thread by means of a driver movable on an orbital path along an orbital path around a knotter assembly and an **envelope element**, such that a loop of two thread sections of the thread end is formed between the knotter assembly and the envelope element;
**b. knotting** the two thread sections by means of the knotter assembly and **releasing** the loop at the envelope element, and
wherein the orbital path comprises a knotting region (V) in which the driver is guided at a first speed, and comprises an envelope region (U) in which the driver is guided at a second speed, and
wherein releasing the loop at the envelope element provides for releasing a latch, which is configured to exert a releasable restoring force in the direction of the driver and/or knotter assembly.

17. The method according to claim 16, wherein
a. feeding a thread end from a thread on a bobbin on the driver movable over the orbital path occurs via a restoring force, and
b. releasing the loop comprises cutting the thread end from the thread.

18. The method according to any of claims 15 or 16, wherein the ratio of the first speed to the second speed is between 1:2 and 1:6.

19. The method according to any of claims 16 to 18, wherein the knotting region (V) encloses an angle of between 5 and 45 degrees of the orbital path, preferably encloses an angle of between 5 and 30 degrees, preferably encloses an angle of 15 degrees.

20. The method according to any of claims 16 to 19, further comprising the steps of:
a. **providing** a device (1) according to claim 1 for knotting a thread end such that a loop is formed;
b. **passing** a strip-shaped substrate through an active region of the driver such that the thread end is laid around the strip-shaped substrate, in particular such that the thread end is laid around the strip-shaped substrate when the driver orbits on the orbital path, wherein the passage of the strip-shaped substrate is arranged to be substantially normal to an orbital path of a driver.

21. A device for producing tampons having a proximal retrieval thread comprising two interlinked thread ends, the device comprising:
a. a device for knotting two thread ends according to any of claims 1 to 15,
b. a **conveying device** for conveying the strip-shaped substrate into an active region of the device for knotting two thread ends, in particular wherein the conveying device is arranged to be substantially normal to an orbital path of a driver for feeding the two threads to a knotter assembly, and
wherein the conveying device for conveying the strip-shaped substrate conveys into the active region of the device for knotting two thread ends such that, during an orbital movement of a driver along an orbital path, the thread is laid around the strip-shaped substrate substantially transversely to the longitudinal axis thereof.

## Revendications

1. Dispositif (1) pour le nouage d'une extrémité de fil de manière à former une boucle, en particulier une extrémité de fil d'un fil de récupération pour tampons, comprenant :
a. un ensemble noueur (3) pour le nouage de deux sections de fil de l'extrémité de fil ;
b. un entraîneur (5) pour alimenter l'ensemble noueur (3) en sections de fil ;
c. un trajet orbital (4) le long duquel l'entraîneur est guidé autour de l'ensemble noueur (3), et
**caractérisé en ce que**
le trajet orbital comporte une zone de nouage (V) dans laquelle l'entraîneur peut se déplacer à une première vitesse, et comporte une zone de retournement (U) dans laquelle l'entraîneur peut se déplacer à une seconde vitesse, et
comprenant en outre un dispositif de maintien (10) en forme de cliquet, qui est conçu pour exercer une force de rappel sur une extrémité de boucle de l'extrémité de fil à nouer.

2. Dispositif selon la revendication 1, comprenant en outre une alimentation en fil pour l'alimentation continue d'une extrémité de fil vers l'entraîneur.

3. Dispositif selon l'une des revendications 1 ou 2, comprenant en outre un élément de coupe pour séparer la boucle de l'extrémité de fil.

4. Dispositif selon l'une des revendications 1 à 3, dans lequel le trajet orbital est un trajet orbital essentiellement circulaire.

5. Dispositif selon l'une des revendications 1 à 4, comprenant une entrée de trajet pour faire passer un substrat en forme de bande à travers la zone fonctionnelle de l'entraîneur, de sorte que l'extrémité de fil est placée autour du substrat en forme de bande, en particulier de sorte que, lors de la rotation de l'entraîneur sur le trajet orbital, l'extrémité de fil est placée autour du substrat en forme de bande.

6. Dispositif selon la revendication 5, comprenant en outre une plaque de guidage pour guider le substrat en forme de bande à travers la zone fonctionnelle de l'entraîneur.

7. Dispositif selon l'une des revendications 1 à 6, comprenant en outre une vérification de noeud pour contrôler le noeud de deux sections de fil liées par l'ensemble noueur (3).

8. Dispositif selon l'une des revendications 1 à 7, dans lequel le trajet orbital est entraînable, en particulier comporte une denture par l'intermédiaire de laquelle le trajet orbital peut être entraîné au moyen d'une courroie crantée, de sorte à pouvoir effectuer une rotation accélérée autour d'un axe de rotation.

9. Dispositif selon l'une des revendications 1 à 7, comprenant un entraînement direct pour l'entraînement de l'entraîneur, en particulier un entraînement direct électrique

10. Dispositif selon l'une des revendications 1 à 9, comprenant en outre un entraînement orbital, en particulier un entraînement orbital à accélération continue, pour entraîner l'entraîneur sur le trajet orbital.

11. Dispositif selon l'une des revendications 1 à 10, dans lequel l'ensemble noueur comprend un mandrin de nouage qui est entraîné en rotation.

12. Dispositif selon l'une des revendications 1 à 11, le dispositif étant configuré pour guider l'entraîneur à travers une zone de nouage à une première vitesse, dans lequel la première vitesse est inférieure à une seconde vitesse à laquelle l'entraîneur est guidé à travers la zone de retournement, en particulier dans lequel la première vitesse a une vitesse maximale qui est entre deux et neuf fois inférieure à une vitesse maximale de la seconde vitesse.

13. Dispositif selon l'une des revendications 1 à 12, dans lequel la première vitesse est comprise entre 300 et 600 °/s et la seconde vitesse est comprise entre 500 et 4000 °/s.

14. Dispositif selon la revendication 13, le dispositif étant configuré pour guider l'entraîneur à travers une zone de nouage à une première vitesse comprise entre 50 et 150 tr/min, en particulier environ 90 tr/min, et pour guider l'entraîneur à travers la zone de retournement à une seconde vitesse comprise entre 200 tr/min et 600 tr/min, en particulier environ 500 tr/min.

15. Dispositif selon l'une des revendications 1 à 14, comprenant en outre une commande d'entraînement pour déplacer l'entraîneur dans la zone de nouage à une première vitesse, et dans une zone de retournement (U) à une seconde vitesse, en particulier dans lequel la commande d'entraînement est conçue pour guider l'entraîneur avec une première vitesse à travers la zone de nouage, de sorte qu'il quitte la zone de nouage après que l'ensemble noueur ait effectué entre un et deux tours, en particulier essentiellement un tour et demi, dans le sens contraire à celui de l'entraîneur.

16. Procédé de nouage d'une extrémité de fil, en particulier deux sections de fil de l'extrémité de fil, de sorte qu'une boucle est formée, laquelle forme un fil de récupération pour tampons, comprenant les étapes consistant à :
a. guider une extrémité de fil d'un fil au moyen d'un entraîneur mobile sur un trajet orbital le long d'un trajet orbital autour d'un ensemble noueur et d'un élément de retournement, de sorte qu'une boucle de deux sections de fil de l'extrémité de fil est formée entre l'ensemble noueur et l'élément de retournement ;
b. nouer les deux sections de fil par l'ensemble noueur et libérer la boucle au niveau de l'élément de retournement, et
dans lequel le trajet orbital comporte une zone de nouage (V) dans laquelle l'entraîneur est guidé à une première vitesse, et comporte une zone de retournement (U) dans laquelle l'entraîneur est guidé à une seconde vitesse, et
dans lequel la libération de la boucle au niveau de l'élément de retournement permet de libérer un verrou qui est configuré pour exercer une force de rappel libérable en direction de l'entraîneur et/ou de l'ensemble noueur.

17. Procédé selon la revendication 16, dans lequel
a. une extrémité de fil d'un fil est alimentée sur une bobine sur l'entraîneur qui peut être déplacé sur le trajet orbital par l'intermédiaire d'une force de rappel, et
b. la libération de la boucle consiste à couper l'extrémité de fil du fil.

18. Procédé selon l'une des revendications 15 ou 16, dans lequel le rapport de la première vitesse sur la seconde vitesse est compris entre 1:2 et 1:6.

19. Procédé selon l'une des revendications 16 à 18, dans lequel la zone de nouage (V) inclus un angle entre 5 et 45 degrés du trajet orbital, de préférence entre 5 et 30 degrés, de préférence 15 degrés.

20. Procédé selon l'une des revendications 16 à 19, comprenant en outre les étapes consistant à :
a. fournir un dispositif (1) selon la revendication 1 pour le nouage d'une extrémité de fil de sorte qu'une boucle est formée ;
b. passer un substrat en forme de bande à travers une zone fonctionnelle de l'entraîneur, de sorte que l'extrémité de fil est placée autour du substrat en forme de bande, en particulier de sorte que, lors de la rotation de l'entraîneur sur le trajet orbital, l'extrémité de fil est placée autour du substrat en forme de bande, le passage du substrat en forme de bande étant essentiellement agencé perpendiculairement à un trajet orbital de l'entraîneur.

21. Dispositif de fabrication de tampons avec un fil de récupération proximal comprenant deux extrémités de fil nouées entre elles, le dispositif comprenant :
a. un dispositif pour le nouage de deux extrémités de fil selon l'une des revendications 1 à 15,
b. un dispositif de transport pour transporter un substrat en forme de bande dans une zone fonctionnelle du dispositif pour le nouage de deux extrémités de fil, en particulier le dispositif de transport étant agencé essentiellement perpendiculairement à un trajet orbital d'un entraîneur pour l'alimentation des deux fils à un ensemble noueur, et
dans lequel le dispositif de transport pour le transport du substrat en forme de bande effectue le transport dans la zone fonctionnelle du dispositif pour le nouage de deux extrémités de fil de sorte que, lors d'un mouvement orbital d'un entraîneur le long d'un trajet orbital, le fil est placé essentiellement transversalement à l'axe longitudinal du substrat en forme de bande autour de celui-ci.
